# EUROPEAN PATENT APPLICATION

(11) **EP 4 130 278 A1**
(43) Date of publication of application: **08.02.2023**
(21) Application number: 21773444.1
(22) Date of filing: 26.03.2021
(51) Int. Cl.: C12N 15/61, C12N 1/15, C12N 1/19, C12N 1/21, C12N 5/10, C12N 9/90, C12N 11/082, C12N 15/63, C12P 19/24

(54) **NOVEL L-RHAMNOSE ISOMERASE**

(30) Priority: 26.03.2020 JP 2020055308
(71) Applicant: National University Corporation Kagawa University, Takamatsu-shi, Kagawa 760-8521 (JP)
(72) Inventor: AKIMITSU, Kazuya, Takamatsu-shi, Kagawa 760-8521 (JP); IZUMORI, Ken, Takamatsu-shi, Kagawa 760-8521 (JP); YOSHIHARA, Akihide, Takamatsu-shi, Kagawa 760-8521 (JP); KATO, Shiro, Takamatsu-shi, Kagawa 760-8521 (JP); MOCHIZUKI, Susumu, Takamatsu-shi, Kagawa 760-8521 (JP); YOSHIDA, Hiromi, Takamatsu-shi, Kagawa 760-8521 (JP); KAMITORI, Shigehiro, Takamatsu-shi, Kagawa 760-8521 (JP)
(74) Representative: TBK
(86) International application number: PCT/JP2021/012989
(87) International publication number: WO 2021/193949

(57) **Abstract**

A highly safe, highly active, and heat-resistant L-rhamnose isomerase and an amino acid-substituted mutant thereof, a microorganism producing them, or a method for producing an aldose or a ketose are provided, all of which are capable of being used in the food industry.

L-Rhamnose isomerase, which is capable of being obtained from a microorganism belonging to the genus Erwinia, has a 48 kDa subunit molecular mass measured by SDS-PAGE, and has the following substrate specificities (A) and (B).
(A) An isomerase activity of recognizing and reacting with a CHO group of C1 and an OH group of C2 of an aldose to convert the CHO group of C1 to an OH group and the OH group of C2 to a CO group, alternatively, recognizing and reacting with an OH group of C1 and a CO group of C2 of a ketose to convert the OH group of C1 to a CHO group and the CO group of C2 to an OH group.
(B) An activity of catalyzing an isomerization reaction between L-rhamnose and L-rhamnulose, between L-lyxose and L-xylulose, between L-mannose and L-fructose, between D-ribose and D-ribulose, between L-talose and L-tagatose, and between D-allose and D-allulose.

## Description

### TECHNICAL FIELD

The present invention relates to a novel L-rhamnose isomerase and a method for producing the same, a microorganism producing the same, a DNA encoding the enzyme and a recombinant vector and a transformed host cell containing the DNA, a L-rhamnose isomerase mutant, and a method for producing a ketose or an aldose by the L-rhamnose isomerase or the mutant.

### BACKGROUND ART

According to the definition of the International Society of Rare Sugars, rare sugars are "sugars that are rarely present in the nature", that is, monosaccharides that are present in a small amount in the nature.

Monosaccharides having four carbon atoms (tetrose) include four types of aldoses, two types of ketoses, and three types of sugar alcohols. Monosaccharides having five carbon atoms (pentose) include eight types of aldoses, four types of ketoses, and four types of sugar alcohols. There are a total of 34 types of monosaccharides having six carbon atoms (hexose), which are 16 types of aldoses, eight types of ketoses, and ten types of sugar alcohols. As types of monosaccharides having seven carbon atoms (heptose), 32 types of aldoheptoses, 16 types of ketoheptoses, and 16 types of heptitols are present, all of which have seven carbon atoms.

For example, for six-carbon sugars (hexose), there are generally six types of aldoses present in large amounts in the nature, which are D-glucose, D-galactose, and D-mannose, and other aldoses such as D-allose are defined as rare sugars. That is, examples of rare sugars among aldoses include L-allose, L-gulose, L-glucose, L-galactose, L-altrose, L-idose, L-mannose, L-talose, D-talose, D-idose, D-altrose, D-gulose, and D-allose. For ketoses, D-fructose is present in large amounts in the nature, whereas other ketoses are not present in the nature in large amounts, and thus are called rare sugars. Examples of rare sugars among ketoses include D-allulose (also known as D-psicose), D-tagatose, D-sorbose, L-fructose, L-allulose (also known as L-psicose), L-tagatose, and L-sorbose.

Recently, the mass production technology for D-allulose (also known as D-psicose), which is the basic raw material for the production of all rare sugars, has been established, making it possible to produce rare sugars that were difficult to obtain. Furthermore, it is thought that D-allulose plays a central role in the production of the other rare sugars such as D-allose by enzyme reaction.

D-allose is an aldose which is an isomer differing from D-glucose only in the direction of the OH group at the C3 position and is a rare monosaccharide also known as an isomer of D-allulose which is a ketose. D-allose is known as a pharmaceutical composition for treating a renal disease selected from acute renal failure and uremia (Patent Document 1), a pharmaceutical product for delaying the onset or progression of dyskinesia caused by amyotrophic lateral sclerosis (Patent Document 2), an antihypertensive agent (Patent Document 3), an agent characterized by being used to inhibit angiogenesis (Patent Document 4), a T lymphocyte proliferation inhibitor (Patent Document 5), or a peritoneal deterioration inhibitor used by being blended with a peritoneal dialysis solution (Patent Document 6), all of which have D-allose as an active ingredient. D-allose is also known as an edible agrochemical (Patent Document 7). Recently, applications have been filed for inventions relating to antitumor effects when being incorporated into renal cell cancer cells (Japanese Patent Application No. 2019-52195) and strong antitumor effects on human urothelial cancer cells (Japanese Patent Application No. 2019-58477). Due to these characteristics, D-allose is attracting attention as a next-generation core material in the fields of pharmaceuticals and agrochemicals, and next to D-allulose, the establishment of the mass production technology for D-allose is desired.

The present inventors developed a technology for producing D-allose from D-allulose by using L-rhamnose isomerase isolated from Pseudomonas stutzeri as a method for producing D-allose using an enzyme produced by microorganisms (Non-Patent Document 1). L-Rhamnose isomerase is an enzyme that catalyzes the reversible isomerization reaction between L-rhamnose and L-rhamnulose, but it was revealed that L-rhamnose isomerase derived from P. stutzeri has broad substrate specificity capable of acting not only between L-rhamnose and L-rhamnulose, but also between L-lyxose and L-xylulose, between L-mannose and L-fructose, between D-gulose and D-sorbose, between D-ribose and D-ribulose, between D-allose and D-allulose, and between L-talose and L-tagatose. Utilizing this broad substrate specificity, it has become possible to produce various rare aldoses and ketoses on Izumoring, focusing on the conversion from D-allulose to D-allose.

### Prior Art

### Patent Documents

Patent Document 1: Japanese Patent No. 5330976
Patent Document 2: Japanese Patent No. 5317055
Patent Document 3: Japanese Patent No. 5158779
Patent Document 4: Japanese Patent No. 4943839
Patent Document 5: Japanese Patent No. 4724824
Patent Document 6: Japanese Patent Application Laid-Open No. 2009-269887
Patent Document 7: Japanese Patent No. 5816871
Patent Document 8: United States Patent No. 10,480,018

### Non-Patent Document

Non-Patent Document 1: J. Ferment. Bioeng. (1997) Vol. 84, p. 319

### SUMMARY

### Technical Problem

According to the condition in which most rare sugar production has become possible today, new challenges have arisen one after another in the rare sugar production. For example, a new challenge for the present inventors is to shift from laboratory-level mass production of the rare sugar D-allose, for which a physiological activity was revealed, to market-scale mass production leading to industrialization. That is, it was thought that first, it was indispensable to select enzymes having a higher thermotolerant activity and a wide range of optimum pH in order to enable efficient mass production of rare sugar.

As described above, in the prior art, rare sugar D-allose is produced using a known L-rhamnose isomerase (EC 5.3.1.14) with pure rare sugar D-allulose as a substrate material. L-Rhamnose isomerase is an enzyme that catalyzes the isomerization reaction from L-rhamnose to L-rhamnulose and can also catalyze the isomerization from L-rhamnulose to L-rhamnose. L-Rhamnose isomerase is also known to act on the isomerization between D-allose and D-allulose. Because isomerases are named based on the substrate showing the highest activity, the substrate specificity varies among the enzymes named L-rhamnose isomerase.

In addition, some of the known enzymes also produce D-altrose, which is an aldose, as a by-product when producing D-allose, and the presence of D-altrose that is a by-product is the cause of the yield decrease of D-allose in a D-allose purification step. Accordingly, the purity of the rare sugar D-allose by mass production has also become one of the challenges.

An object of the present invention is to provide a novel L-rhamnose isomerase which is derived from a microorganism approved for use when producing foods and regarded to be non-toxic, which is highly active, and which is capable of isomerizing from D-allulose to D-allose at a high yield, a microorganism having the enzyme, and a production method using this enzyme. Solution to Problem

The present inventors sampled the soil from various regions and isolated a microorganism therefrom to continue searching for a microorganism having L-rhamnose isomerase activity by paying attention to the species of bacteria which are regarded to be almost non-toxic and are on the list approved for use as foods not only in Japan but also in Europe and the United States.

As a result, they found a microorganism belonging to the genus Erwinia which produces a novel L-rhamnose isomerase from many isolated strains. Erwinia billingiae which is this microorganism belonging to the genus Erwinia produces a novel L-rhamnose isomerase having a high activity and high heat resistance.

The novel L-rhamnose isomerase derived from this microorganism catalyzes the isomerization reaction between an aldose and a corresponding ketose, and has an isomerase activity between an aldose and a ketose to recognize and react with a CHO group of C1 and an OH group of C2 of an aldose to convert the CHO group of C1 to an OH group and the OH group of C2 to a CO group, alternatively, recognize and react with an OH group of C1 and a CO group of C2 of a ketose to convert the OH group of C1 to a CHO group and the CO group of C2 to an OH group. In addition, the L-rhamnose isomerase is suitable for production of D-allose because D-altrose, which is a by-product, is not produced when producing D-allose using D-allulose as a substrate.

That is, the present invention relates to an L-rhamnose isomerase described in the following (1) to (4), and a microorganism described in the following (5).
(1) An L-rhamnose isomerase derived from a microorganism belonging to the genus Erwinia, in which a subunit molecular mass measured by SDS-PAGE is 48 kDa, and the L-rhamnose isomerase has the following substrate specificities (A) and (B).
   (A) An isomerase activity of recognizing and reacting with a CHO group of C1 and an OH group of C2 of an aldose to convert the CHO group of C1 to an OH group and the OH group of C2 to a CO group, alternatively, recognizing and reacting with an OH group of C1 and a CO group of C2 of a ketose to convert the OH group of C1 to a CHO group and the CO group of C2 to an OH group.
   (B) An activity of catalyzing an isomerization reaction between L-rhamnose and L-rhamnulose, between L-lyxose and L-xylulose, between L-mannose and L-fructose, between D-ribose and D-ribulose, between L-talose and L-tagatose, and between D-allose and D-allulose.
(2) The L-rhamnose isomerase according to (1), in which the L-rhamnose isomerase has the following physicochemical properties (C) and (D).
   (C) An optimum reaction pH is 9.
   (D) An optimum reaction temperature is 70°C.
(3) The L-rhamnose isomerase according to (1) or (2), in which the microorganism belonging to the genus Erwinia is Erwinia billingiae.
(4) The L-rhamnose isomerase according to any one of (1) to (3), in which the microorganism belonging to the genus Erwinia is Erwinia billingiae strain GuaL218-3 that is internationally deposited in the NITE Patent Microorganisms Depositary as an accession number NITE BP-03142.

The present invention also relates to a protein described in the following (5) to (7), a DNA, a recombinant vector, or a transformed host cell described in the following (8) to (11), or a microorganism described in the following (12).
(5) A protein comprising an amino acid sequence set forth in SEQ ID NO: 1.
(6) A protein that is an amino acid-substituted mutant of a protein consisting of an amino acid sequence set forth in SEQ ID NO: 1, in which the mutant has 80% or more identity with the amino acid sequence set forth in SEQ ID NO: 1, has amino acid substitution at least one site selected from K3, L4, I5, Y9, E10, L11, Y16, D18, V19, I21, V23, Q25, V26, M27, T28, G32, I33, R46, N52, E54, R68, H73, A77, I79, E80, K81, M83, A89, D102, T103, E106, D108, A109, E111, Q113, S116, H117, Q124, H125, K126, S134, S148, D151, K152, G153, C162, I168, H171, P179, V186, L193, I195, L198, A199, E202, A205, S206, V211, F216, D217, A218, S219, C245, L246, A250, T257, T275, V280, L300, T310, A313, N315, K316, N319, K320, A345, S357, D358, Q359, R361, K362, L365, E366, A371, L375, V387, A390, W391, L393, H395, V397, D400, A401, S402, S405, E406, H409, Q412, Q413, T414, R416, L417, and 419 of the amino acid sequence set forth in SEQ ID NO: 1, has the following L-rhamnose isomerase activities (A) and (B), and has a higher L-rhamnose isomerase activity at an optimum temperature than the protein consisting of the amino acid sequence set forth in SEQ ID NO: 1.
   (A) An isomerase activity of recognizing and reacting with a CHO group of C1 and an OH group of C2 of an aldose to convert the CHO group of C1 to an OH group and the OH group of C2 to a CO group, alternatively, recognizing and reacting with an OH group of C1 and a CO group of C2 of a ketose to convert the OH group of C1 to a CHO group and the CO group of C2 to an OH group.
   (B) An activity of catalyzing an isomerization reaction between L-rhamnose and L-rhamnulose, between L-lyxose and L-xylulose, between L-mannose and L-fructose, between D-ribose and D-ribulose, between L-talose and L-tagatose, and between D-allose and D-allulose.
(7) A protein that is an amino acid-substituted mutant of a protein consisting of an amino acid sequence set forth in SEQ ID NO: 1, in which the mutant has 78% or more identity with the amino acid sequence set forth in SEQ ID NO: 1, has amino acid substitution at least one site selected from K3, L4, I5, Y9, E10, L11, Y16, D18, V19, I21, V23, E24, Q25, V26, M27, T28, G32, I33, R46, N52, E54, R68, H73, A77, I79, E80, K81, A82, M83, S84, A89, K90, I97, D102, T103, E106, D108, A109, E111, Q113, S116, H117, E120, Q124, H125, K126, S134, P139, L140, S148, A150, D151, K152, G153, I154, C162, R167, I168, H171, P179, V186, L193, I195, L198, A199, E202, A205, S206, E210, V211, K215, F216, D217, A218, S219, C245, L246, A250, T254, T257, T275, V280, R282, L300, T310, A313, N315, K316, N319, K320, A345, S357, D358, Q359, R361, K362, L363, L365, E366, Y369, A371, A374, L375, S381, V387, A390, W391, L393, H395, V397, D400, A401, S402, S405, E406, H409, Q412, Q413, T414, R416, L417, and 419 of the amino acid sequence set forth in SEQ ID NO: 1, has the following L-rhamnose isomerase activities (A) and (B), and has a higher L-rhamnose isomerase activity ratio (T70/T50) at a reaction temperature of 70°C and 50°C, a higher residual activity after incubation at 60°C for one hour, or a higher L-rhamnose isomerase activity ratio (T80/optimum temperature) at a reaction temperature of 80°C and an optimum temperature, than the protein consisting of the amino acid sequence set forth in SEQ ID NO: 1.
   (A) An isomerase activity of recognizing and reacting with a CHO group of C1 and an OH group of C2 of an aldose to convert the CHO group of C1 to an OH group and the OH group of C2 to a CO group, alternatively, recognizing and reacting with an OH group of C1 and a CO group of C2 of a ketose to convert the OH group of C1 to a CHO group and the CO group of C2 to an OH group.
   (B) An activity of catalyzing an isomerization reaction between L-rhamnose and L-rhamnulose, between L-lyxose and L-xylulose, between L-mannose and L-fructose, between D-ribose and D-ribulose, between L-talose and L-tagatose, and between D-allose and D-allulose.
(8) A DNA encoding the protein according to any one of (5) to (7).
(9) A DNA comprising a base sequence set forth in SEQ ID NO: 2.
(10) A recombinant vector comprising the DNA according to (8) or (9).
(11) A transformed host cell which is transformed by the recombinant vector according to (10).
(12) Erwinia billingiae GuaL218-3 which is internationally deposited in the NITE Patent Microorganisms Depositary as an accession number NITE BP-03142, in which the Erwinia billingiae GuaL218-3 produces the L-rhamnose isomerase according to any one of (1) to (4).

The present invention also relates to an immobilized protein described in the following (13) to (16).
(13) An immobilized protein, in which the L-rhamnose isomerase according to any one of (1) to (4) or the protein according to any one of (5) to (7) is immobilized on a carrier.
(14) An immobilized protein, in which the L-rhamnose isomerase according to any one of (1) to (4) is immobilized on a carrier in a form of a crude enzyme present in a bacterial cellextract or the protein according to any one of (5) to (7) is immobilized on a carrier in a form of a crude protein present in a bacterial cell-disrupted product of a transformed host cell.
(15) The immobilized protein according to (13) or (14), in which the carrier is an ion exchange resin or a synthetic adsorbent.
(16) The immobilized protein according to (15), in which the carrier is WA30, FPA54, or FPA95.

The present invention also relates to a method for producing an L-rhamnose isomerase or a method for producing a ketose or an aldose as described in the following (17) to (19).
(17) A method for producing L-rhamnose isomerase, the method comprising culturing a microorganism belonging to the genus Erwinia which produces the L-rhamnose isomerase according to any one of (1) to (4) or the transformed host cell according to (11) in a culture medium to accumulate and collect the L-rhamnose isomerase in a microorganism bacterial cell.
(18) The method for producing L-rhamnose isomerase according to (17), in which the culture medium is a mineral salt culture medium to which a L-rhamnose is added.
(19) A method for producing a ketose or an aldose, the method comprising causing the L-rhamnose isomerase according to any one of (1) to (4), the protein according to any one of (5) to (7), or the immobilized protein according to any one of (13) to (16) to act on a solution containing one or more selected from aldoses and ketoses to generate and collect a corresponding ketose or aldose.

### Advantageous Effects of Invention

The L-rhamnose isomerase of the present invention is characterized in that it has particularly high heat resistance and a high activity as compared to conventional L-rhamnose isomerases derived from microorganisms. For example, a conventional L-rhamnose isomerase derived from Pseudomonas stutzeri has an optimum temperature of 60°C, whereas the enzyme of the present invention has a high optimum temperature of 70°C, and also has thermal stability in which a residual activity after heat treatment at 60°C for 10 minutes is 80% or more, which makes the enzyme suitable for use in industrial production. The excellent heat resistance of this enzyme is a characteristic that is capable of being further enhanced by creating an amino acid-substituted mutant of this enzyme.

In addition, when a substrate is 100 mM D-allulose, the enzyme of the present invention has a conversion activity to D-allose as high as 2.26 U per unit protein at 60°C, which makes the mass production of D-allose possible by the present invention. In particular, when a substrate is D-allulose, the enzyme of the present invention converts it only to D-allose and does not generate D-altrose as a by-product, thereby increasing the yield accordingly.

### BRIEF DESCRIPTION OF DRAWINGS

Fig. 1 illustrates a graph of a comparison of the generation amount of D-allose between the present crude enzyme (derived from the strain GuaL218-3) and a control crude enzyme (derived from the strain AgM30).
Fig. 2 illustrates the results of SDS-PAGE to confirm the molecular mass of the present enzyme. In the drawing, the unit of the numbers on the left is kDa.
Fig. 3 illustrates graphs of the optimum pH of the present enzyme and the control enzyme.
Fig. 4 illustrates graphs of the pH stability of the present enzyme and the control enzyme after 24 hours.
Fig. 5 illustrates graphs of the optimum temperature of the present enzyme and the control enzyme.
Fig. 6 illustrates graphs of the temperature stability of the present enzyme and the control enzyme after 10-minute incubation.
Fig. 7 illustrates graphs of the substrate specificity of the present enzyme and the control enzyme.

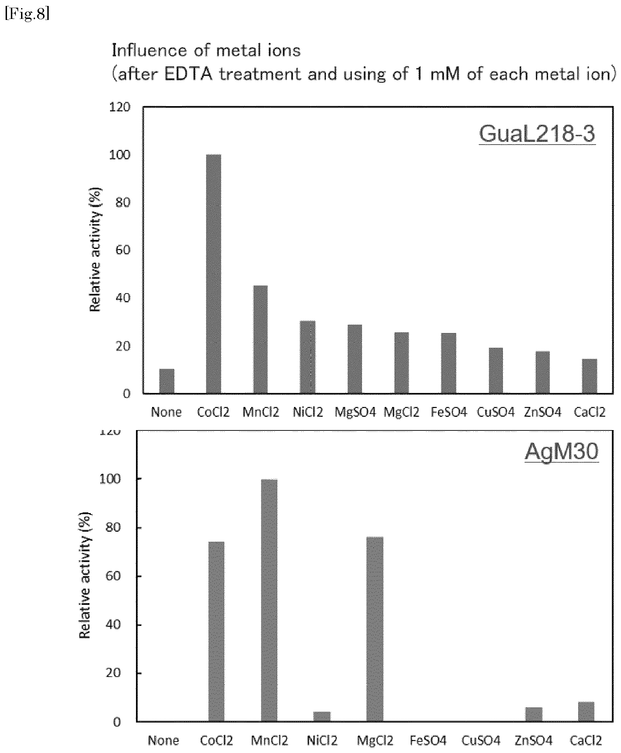

Fig. 8 illustrates graphs of the influence of metal ions on the present enzyme and the control enzyme.
Fig. 9 illustrates a graph of the relative value of the activity expression rate of an immobilized enzyme of the present enzyme by an immobilization carrier.
Fig. 10 illustrates the results of SDS-PAGE of a recombinant enzyme expressed in Escherichia coli.
Fig. 11 illustrates the results of HPLC analysis of a reaction solution in which a conversion reaction from D-allulose to D-allose by a recombinant enzyme was caused.

### DESCRIPTION OF EMBODIMENTS

The present invention relates to L-rhamnose isomerase which is capable of being isolated from a microorganism belonging to the genus Erwinia and has characteristic properties of a high activity and heat resistance.

The L-rhamnose isomerase of the present invention has an isomerase activity of recognizing and reacting with a CHO group of C1 and an OH group of C2 of an aldose and converting the CHO group of C1 to an OH group and the OH group of C2 to a CO group to obtain a ketose, alternatively, recognizing and reacting with an OH group of C1 and a CO group of C2 of a ketose and converting the OH group of C1 to a CHO group and the CO group of C2 to an OH group to obtain an aldose.

A ketose referred to in the present invention means a ketohexose of six-carbon sugars or a ketopentose of five-carbon sugars having a ketose structure. Ketohexoses include allulose (also known as psicose), sorbose, tagatose, and fructose, and ketopentoses include ribulose and xylulose.

An aldose referred to in the present invention means an aldohexose of six-carbon sugars or an aldopentose of five-carbon sugars having an aldose structure. Aldohexoses include glucose, allose, altrose, gulose, idose, talose, galactose, and mannose, and aldopentoses include ribose, arabinose, xylose, and lyxose. D- or L- means the D-form and the L-form thereof.

The L-rhamnose isomerase of the present invention acts on L-rhamnose, L-lyxose, L-mannose, D-ribose, L-talose, and D-allose, and has a broad substrate specificity capable of catalyzing the conversions between L-rhamnose-L-rhamnulose, between L-lyxose and L-xylulose, between L-mannose and L-fructose, between D-ribose and D-ribulose, between L-talose and L-tagatose, and between D-allose and D-allulose.

The L-rhamnose isomerase of the present invention can be prepared by culturing a microorganism belonging to the genus Erwinia and having the ability to produce an L-rhamnose isomerase, and isolating an L-rhamnose isomerase from the bacterial cells grown in a culture medium. As the microorganism belonging to the genus Erwinia, Erwinia billingiae strain GuaL218-3 and mutants thereof can be advantageously utilized. In filing the present application, the strain GuaL218-3 was internationally deposited on February 28, 2020 in the National Institute of Technology and Evaluation, NITE Patent Microorganisms Depositary located at 2-5-8 Kazusa Kamatari, Kisarazu, Chiba, Japan, and was received as the accession number NITE BP-03142. Thereafter, the strain GuaL218-3 was officially deposited on February 28, 2020 based on the Budapest Treaty as the accession number NITE BP-03142.

The L-rhamnose isomerase of the present invention is extracted from a culture liquid of bacterial cells by centrifugation after aeration culture of L-rhamnose isomerase-producing bacteria belonging to the genus Erwinia in a mineral salt medium to which L-rhamnose has been added. The collected bacterial cells were washed using a 10 mM tris-HCl buffer (pH 7.5), thereafter suspended in 10 ml of a 10 mM tris-HCl buffer (pH 7.5) to disrupt the cells by enzyme treatment with a lysozyme added which is a lytic enzyme or disrupt the cells with an ultrasonic homogenizer while cooling the bacterial cell suspension in ice water. This disrupted product is centrifuged, and the supernatant is used as a crude enzyme solution.

The activity of L-rhamnose isomerase in the crude enzyme solution before purification can be confirmed by measuring the generation amount of D-allose using D-allulose as a substrate.

The enzyme activity for converting from D-allose to D-allulose, which is the reverse reaction, is also measured under the same conditions. These conversion reactions are usually performed under the following conditions. The substrate concentration is 1% to 60% (w/v) and is desirably about 5% to 50% (w/v), the reaction temperature is 30°C to 80°C and is desirably about 50°C to 70°C, the reaction pH is 6 to 11 and is desirably about 8 to 11, and the reaction time can be appropriately selected, but the range of 4 to 20 hours is usually selected in the case of a batch reaction.

The crude enzyme solution can be purified by sequential ion exchange chromatography and hydrophobic chromatography to isolate a purified enzyme. SDS-PAGE (12.5% gel concentration) is performed to confirm the purification of the enzyme, thereby confirming that a single band is obtained and the apparent molecular mass.

The L-rhamnose isomerase of the present invention purified as described above has the approximately 48 kDa subunit molecular mass by SDS-PAGE and is a metalloenzyme in which the degree of activation is regulated by metal ions. The reaction with the substrate may be performed in the presence of metal ions selected from the group consisting of manganese, cobalt, nickel, magnesium, iron, copper, zinc, and calcium at the concentration of 0.5 to 5 mM.

The L-rhamnose isomerase of the present invention has a predetermined amino acid sequence. Examples thereof include a protein having an amino acid sequence set forth in SEQ ID NO: 1, and a protein having an amino acid sequence homologous thereto and maintaining an equivalent L-rhamnose isomerase activity. The homologous amino acid sequence means an amino acid sequence 75% or more, 78% or more, and 80% or more, preferably 85% or more, more preferably 90% or more, and further more preferably 95% or more identical to the amino acid sequence of SEQ ID NO: 1, for example.

The identity (%) of two amino acid sequences or two nucleotide sequences can be determined by the following procedure, for example. First, two sequences are arranged so that the optimal comparison is possible. At this time, for example, a gap may be introduced into the first sequence to optimize the alignment with the second sequence. When a molecule (amino acid residue or nucleotide) at a specific position in the first sequence and a molecule at a position corresponding thereto in the second sequence are the same, the molecules at the position are said to be identical. The identity of the two sequences is a function of the number of the same positions common to these two sequences (that is, identity (%) = number of same positions/total number of positions x 100), and the number and size of the gap needed for the optimization of the alignment is preferably taken into consideration.

In addition, the comparison of the two sequences and determination of the identity can be realized using a mathematical algorism. Specific examples of the mathematical algorism usable for sequence comparison include, but not limited to, the algorism described in Karlin and Altschul (1990) Proc. Natl. Acad. Sci. USA 87: 2264-68 and altered in Karlin and Altschul (1993) Proc. Natl. Acad. Sci. USA 90: 5873-77. Such an algorism has been incorporated in the NBLAST program and XBLAST program (version 2.0) described in Altschul et al., (1990) J. Mol. Biol. 215: 403-10. A nucleotide sequence equivalent to the nucleic acid molecule of the present invention may be obtained, for example, by performing BLAST nucleotide search with score=100 and wordlength=12 by using the NBLAST program.

A DNA of the present invention is a gene encoding the above-mentioned protein and has a predetermined base sequence. Examples thereof include a DNA sequence encoding the amino acid sequence set forth in SEQ ID NO: 1, a nucleotide sequence set forth in SEQ ID NO: 2, and a DNA sequence having a nucleotide sequence homologous to the nucleotide sequence set forth in SEQ ID NO: 2 and encoding a protein maintaining an L-rhamnose isomerase activity equivalent to that of the protein of SEQ ID NO: 1. The homologous nucleotide sequence means a sequence 75% or more, 78% or more, and 80% or more, preferably 85% or more, more preferably 90% or more, and further more preferably 95% or more identical to the sequence of SEQ ID NO: 2, for example.

The DNA of the present invention can be inserted into an autonomously replicable and appropriate vector to obtain a recombinant vector. The recombinant vector is composed of a DNA and an autonomously replicable vector, and can be prepared relatively easily by the conventional recombination DNA technology if a DNA can be obtained. An appropriate vector is selected depending on the intended use such as cloning or protein expression, or depending on host cells. Examples of such a vector include plasmid vectors such as pBR322, pUC18, pUB110, pTZ4, pC194, pHV14, TRp7, YEp7, and pBS7, and phage vectors such as λgt·λC, λgt·λB, ρ11, cp 1, and cp 105.

The recombinant vector thus obtained can be introduced into appropriate host cells including Escherichia coli, Bacillus subtilis, Actinomycetes, and yeast. For an introduction method, a known method such as a calcium phosphate coprecipitation method, an electroporation method, a lipofection method, and a microinjection method is used. A colony hybridization method or the like is applied to acquire transformed host cells.

A method of producing an L-rhamnose isomerase and a protein having the L-rhamnose isomerase activity of the present invention is not particularly limited, and a known method can be adopted. Specifically, the L-rhamnose isomerase of the present invention can be produced by collecting a protein having an L-rhamnose isomerase activity from a cultured product obtained by culturing, in a nutrient medium, a microorganism of the present invention having an ability to produce an L-rhamnose isomerase or host cells transformed with a DNA encoding the protein of the present invention having the L-rhamnose isomerase activity. For a culturing method, a known method can be used, and any of liquid culture or solid culture can be used, for example.

After the bacterial cells are cultured in such methods, the enzyme and the protein of the present invention are purified and recovered. A method of purification and recovery of the enzyme and the protein can be selected freely from known methods and performed. For example, in the case of recovery from a culture medium, separation and purification can be performed by removing insoluble matter by subjecting the culture supernatant to, for example, filtration, centrifugation treatment, or the like, and thereafter using, in combination, enrichment through an ultrafiltration membrane, salt precipitation such as ammonium sulfate precipitation, dialysis, and various chromatographies such as an ion exchange resin, and the like. Furthermore, in the case of recovery from the bacterial cells, for example, the bacterial cells are disrupted by lytic enzyme treatment, ultrasonic treatment, or the like to perform the similar separation and purification thereafter.

Furthermore, the L-rhamnose isomerase of the present invention can be used in the form of an immobilized enzyme as in the case of other isomerases. The immobilized enzyme having a high activity can be obtained by various immobilization methods. Using the immobilized enzyme makes it possible to continuously perform a mass isomerization reaction. The immobilized enzyme can be obtained by using known immobilization means, for example, a carrier binding method, a crosslinking method, a gel encapsulation method, and a microcapsulation method. Furthermore, the carrier may be any known carrier.

In one aspect of a method of immobilizing the L-rhamnose isomerase of the present invention, immobilization is performed using a crude enzyme solution. The L-rhamnose isomerase can be immobilized by adding an L-rhamnose isomerase-containing crude enzyme solution obtained by ultrasonic treatment of the bacterial cell suspension to an ion exchange resin or the like and causing binding at a low temperature.

It is required to disrupt the bacterial cell wall to take out the L-rhamnose isomerase from the bacterial cells, and as described above, there are disruption methods by ultrasonic treatment and treatment with an enzyme such as lysozyme. It was found that a crude enzyme solution obtained by the ultrasonic treatment is rich in an L-rhamnose isomerase having an activity. As a carrier on which the enzyme from the crude enzyme solution is immobilized, any known immobilization carrier can be used, but ion exchange resins, sodium alginate, synthetic adsorbents, and the like are frequently used because they are convenient.

Among ion exchange resins, as a basic anion exchange resin, any of a strongly basic anion exchange resin or a weakly basic anion exchange resin can be used, for example. Examples of the strongly basic anion exchange resin include SA20A and PA418 (manufactured by Mitsubishi Chemical Corporation) and the like, and examples of the weakly basic anion exchange resin include WA30 (manufactured by Mitsubishi Chemical Corporation), FPA54 and FPA95 (manufactured by Organo), and the like. Examples of the synthetic adsorbent include XAD7HP (manufactured by Organo) and the like.

When the immobilized enzyme is produced using the weakly basic anion exchange resin as a carrier, the immobilized L-rhamnose isomerase can be easily eluted after the reaction, making it possible to very easily perform the regeneration of the immobilized enzyme, which is good production efficiency.

The L-rhamnose isomerase of the present invention can be produced by collecting a protein having an L-rhamnose isomerase activity from a cultured product obtained by culturing, in a nutrient medium, a microorganism of the present invention having an ability to produce the L-rhamnose isomerase or host cells transformed with a DNA encoding the protein having the L-rhamnose isomerase activity. For a culturing method, a known method can be used, and any of liquid culture or solid culture can be used, for example.

After culturing the bacterial cells in the culture medium, the L-rhamnose isomerase of the present invention is purified and recovered. A method of purification and recovery of the protein can be selected freely from known methods and performed. For example, in the case of recovery from a culture liquid, separation and purification can be performed by removing insoluble matter by subjecting the culture supernatant to, for example, filtration, centrifugation treatment, or the like, and thereafter using, in combination, enrichment through an ultrafiltration membrane, salt precipitation such as ammonium sulfate precipitation, dialysis, and various chromatographies such as an ion exchange resin, and the like. Furthermore, in the case of recovery from the bacterial cells, for example, the bacterial cells are disrupted by lytic enzyme treatment, ultrasonic treatment, or the like to perform the similar separation and purification thereafter.

The purified L-rhamnose isomerase or the immobilized enzyme of the present invention is used to act on a solution containing one or more selected from aldoses or ketoses that can serve as a substrate to generate the corresponding ketoses or aldoses, thereby making it possible to produce them. Since the L-rhamnose isomerase of the present invention has a higher activity for D-allulose than conventional L-rhamnose isomerases, a large amount of D-allose which is a rare sugar can be produced in the presence of D-allulose that can be used as a substrate.

Furthermore, an enzyme having a higher D-allulose isomerase activity at the optimum temperature and an enzyme having higher thermostability than an amino acid substitution-free wild-type enzyme can be obtained by introducing mutation into the gene of the L-rhamnose isomerase of the present invention and substituting the corresponding amino acid residue with another amino acid residue by a site-directed mutagenesis operation to prepare various amino acid-substituted mutants. The thermostability is evaluated by four indices, that is, whether the optimum temperature, the D-allulose isomerase activity ratio (T70/T50) at the reaction temperature of 70°C and 50°C, the residual activity after incubation at 60°C for 1 hour, or the D-allulose isomerase activity ratio (T80/optimum temperature) at the reaction temperature of 80°C and the optimum temperature, is high.

Even when the L-rhamnose isomerase of the present invention is a mutant with the amino acid sequence identity of about 78%, a mutant can be obtained, the mutant maintaining the enzyme activity, and having, as compared to the wild-type enzyme of SEQ ID NO: 1, a higher optimum temperature, a higher D-allulose isomerase activity ratio (T70/T50) at the reaction temperature of 70°C and 50°C, a higher residual activity after incubation at 60°C for 1 hour, or a higher D-allulose isomerase activity ratio (T80/optimum temperature) at the reaction temperature of 80°C and the optimum temperature.

Regarding the design of the amino acid-substituted mutant, a method for creating a phylogenetic tree disclosed in Patent Document 8 was referred to.

All DNA sequences encoding enzymes having an activity similar to that of the L-rhamnose isomerase of the present invention set forth in SEQ ID NO: 1 or putative enzymes are extracted, and using the method of Patent Document 8, a phylogenetic tree that is phylogenetically derived from the DNA sequences of the phylogenetic origin as common ancestral DNA is produced. Then, the amino acid sequences encoded by the obtained DNA sequences having the same lineage as that of SEQ ID NO: 1 of the present invention in the phylogenetic tree are compared. Amino acids and the like corresponding to the different sites in SEQ ID NO: 1 are changed by base substitution by site-directed mutagenesis to produce various L-rhamnose isomerase mutants while focusing on the amino acid sequences of the portions at which the sequences are different and according to the presumption that sites different in amino acid residue are less involved in the enzyme activity. Among them, amino acid-substituted mutants having a higher D-allulose isomerase activity at the optimum temperature and higher thermostability than the wild-type enzyme are selected.

In addition, the amino acid-substituted mutants of the present invention exceptionally include a mutant in which one amino acid residue has been added to the amino acid of the 418th position of the C-terminal.

A site-directed mutagenesis method can be performed by an arbitrary method such as an inverse PCR method, an annealing method (edited by Muramatsu et al., "Revised 4th Edition New Genetic Engineering Handbook", Yodosha, p. 82-88), or the like, for example. If necessary, it is possible to also use various commercially available site-directed mutagenesis kits such as QuickChange II Site-Directed Mutagenesis Kit and QuickChange Multi Site-Directed Mutagenesis Kit of Stratagene.

The site-directed mutagenesis can most commonly be performed using a mutation primer containing nucleotide mutation to be introduced. Such a mutation primer may be designed so as to anneal to a region containing a nucleotide sequence encoding a substitution targeting amino acid residue to be altered and include a base sequence having a nucleotide sequence (codon) encoding an amino acid residue after alteration instead of the nucleotide sequence (codon) encoding an amino acid residue to be altered.

The amino acid-substituted mutant means a mutant in which amino acids in the original sequence are substituted with different amino acids. Although the substitution is not particularly limited and it may be conservative substitution or non-conservative substitution, the substitution in a preferred aspect of the present invention is conservative substitution. The conservative substitution includes a substitution between amino acids having the same properties (basic, acidic, or neutral) or the same polarities (hydrophilic or hydrophobic) in the basic to basic, acidic to acidic, or polar to polar manner, or between aromatic amino acids or between aliphatic amino acids.

The conservative substitution is performed, for example, within groups of basic amino acids (Arg, Lys, His), acidic amino acids (Glu, Asp), neutral non-polar amino acids (Gly, Ala, Val, Leu, Ile, Met), aliphatic amino acids (Ala, Val, Leu, Ile, Met), polar amino acids (Gln, Asn, Ser, Thr), aromatic amino acids (Phe, Trp, Tyr), and the like.

On the other hand, the non-conservative substitution means exchange with an amino acid which is a member other than those of the above-mentioned groups. For example, Cys is deleted or substitution with another amino acid is caused to prevent folding in a protein in a tertiary structure. Alternatively, an amino acid is substituted in order to keep a hydrophilicity/hydrophobicity balance or enhance the degree of hydrophilicity to facilitate synthesis while considering a hydropathy index (J. Mol. Biol. (1982) Vol. 157, p. 105-132) of an amino acid which is an index of hydrophobicity/hydrophilicity relating to an amino acid.

Furthermore, there are a substitution to an amino acid having less steric hindrance than the original amino acid, and a substitution from an amino acid having a charge to an amino acid not having a charge.

The substitution may be conservative substitution or non-conservative substitution. Examples thereof include, but not limited to, a substitution of Ala by Ser or Thr, a substitution of Arg by Gln, His, or Lys, a substitution of Asn by Glu, Gln, Lys, His, or Asp, a substitution of Asp by Asn, Glu, or Gln, a substitution of Cys by Ser or Ala, a substitution of Gln by Asn, Glu, Lys, His, Asp, or Arg, a substitution of Glu by Asn, Gln, Lys, or Asp, a substitution of Gly by Pro, a substitution of His by Asn, Lys, Gln, Arg, or Tyr, a substitution of Ile by Leu, Met, Val, or Phe, a substitution of Leu by Ile, Met, Val, or Phe, a substitution of Lys by Asn, Glu, Gln, His, or Arg, a substitution of Met by Ile, Leu, Val, or Phe, a substitution of Phe by Trp, Tyr, Met, Ile, or Leu, a substitution of Ser by Thr or Ala, a substitution of Thr by Ser or Ala, a substitution of Trp by Phe or Tyr, a substitution of Tyr by His, Phe, or Trp, and a substitution of Val by Met, Ile, or Leu. With respect to the substitution of these amino acid residues, the classification of residues which can be substituted an example, and amino acid residues which can be substituted are not limited to this classification.

In the wild-type enzyme having the amino acid sequence of SEQ ID NO: 1, the production is performed from a mutant in which an amino acid at one site has been substituted to many multiple mutants in which amino acids at 90 sites have been substituted, and enzyme activity and heat resistance tests are performed to select 58 mutants. Among them, 13 mutants out of the 58 mutants are confirmed to have a higher D-allulose isomerase activity at the optimum temperature than the wild-type enzyme without amino acid substitution.

In addition, 52 mutants out of the 58 mutants are confirmed to have a higher D-allulose isomerase activity ratio (T70/T50) at the reaction temperature of 70°C and 50°C than the wild-type enzyme, 17 mutants out of the 58 mutants are confirmed to have a higher residual activity after incubation at 60°C for 1 hour than the wild-type enzyme, and 56 mutants out of the 58 mutants are confirmed to have a higher D-allulose isomerase activity ratio (T80/optimum temperature) at the reaction temperature of 80°C and the optimum temperature than the wild-type enzyme.

The L-rhamnose isomerase of the present invention is also excellent from the viewpoint of obtaining a lot of mutants having higher heat resistance than the wild-type L-rhamnose isomerase.

### [Examples]

The present invention will be described in detail below through experiments, but the present invention is not limited by the following examples.

### <Experiment 1: Origin and identification of strain>

The present inventors and the like inoculated a lot of bacteria isolated by screening in a liquid medium to which L-rhamnose had been added to conduct shaking culture, and measured the generation amount of L-rhamnulose with L-rhamnose as a substrate to measure the L-rhamnose isomerase activity.

In this manner, a microorganism strain GuaL218-3 was found as a strain having the highest activity, and it was revealed that the strain GuaL218-3 belonged to Erwinia by system analysis based on the homology to a 16SrRNA gene base sequence.

### Identification of strain

### (1) Homology to 16SrRNA gene base sequence

A 1 to 500 bp region of the 16SrRNA gene was amplified and analyzed, and the nucleotide sequences of the region were identified.

### (2) Homology search

The search of the homology of the nucleotide sequence of the 16SrRNA gene region of the strain to the nucleotide sequence database of known species of bacteria regarded as the reference strain was performed by BLAST search program (Japan DNA Data Bank). The microorganism of the strain GuaL218-3 was determined to be Erwinia billingiae from the name of homologous strain which has 98% or more homology in the nucleotide sequence of the analyzed region.

The strain GuaL218-3 was internationally deposited on February 28, 2020 at the National Institute of Technology and Evaluation, Patent Microorganisms Depositary Center, and was internationally deposited as the accession number NITE BP-03142.

### <Experiment 2: Culture of Erwinia billingiae strain GuaL218-3 (accession number NITE BP-03142)>

A 1% (v/v) seed culture of the Erwinia billingiae strain GuaL218-3 (accession number: NITE BP-03142) was aseptically added to a mineral salt liquid medium with 1.0% L-rhamnose as a carbon source and ammonium sulfate as a nitrogen source to perform culturing at 30°C for 24 hours while aerating and stirring.

As a control strain for comparison with the strain of the present invention, an Arthrobacter globiformis strain M30 (NITE BP-1 111) (hereinafter referred to as "strain AgM30") was cultured in the same culture medium and culture conditions as the strain GuaL218-3.

### <Experiment 3: Preparation of crude enzyme>

Bacterial cells were collected by centrifugation from the respective liquid cultures of the strain GuaL218-3 and the strain AgM30. The respective collected bacterial cells were washed using a 10 mM Glycine-NaOH buffer (pH 9.0). Subsequently, the bacterial cells were suspended in 10 mL of Glycine-NaOH buffer (pH 9.0), and thereafter, cell disruption was caused by an ultrasonic homogenizer (Japan Emerson) while cooling the resulting bacterial cell suspension in ice water. The disrupted product was centrifuged at 12,000 rpm for 20 minutes to obtain the centrifugation supernatant as the respective crude enzymes.

### <Experiment 4: Production of D-allose by crude enzyme>

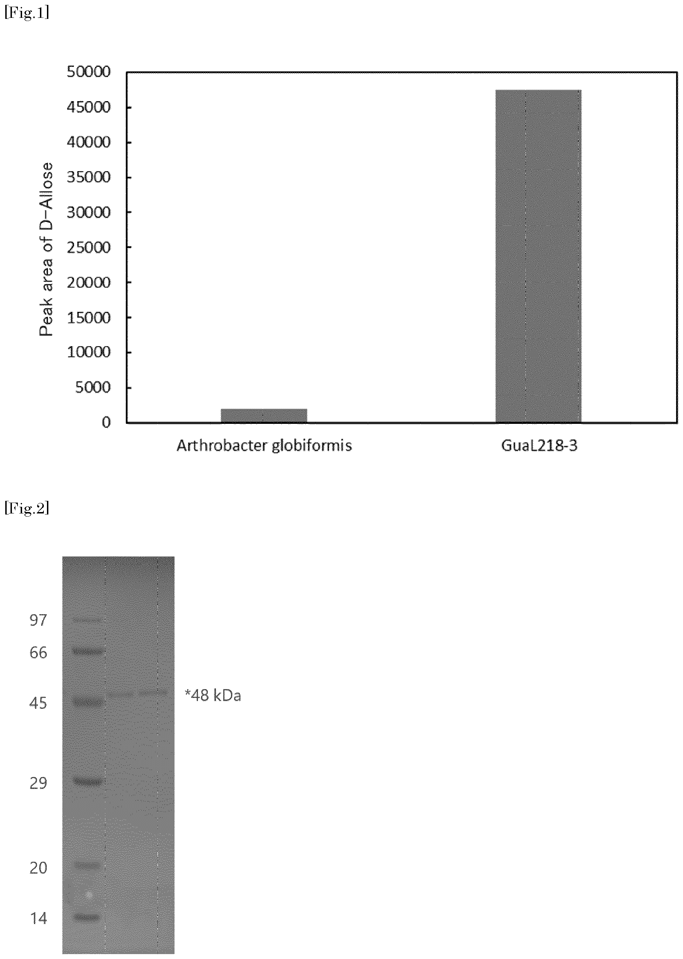

Using the two crude enzyme solutions thus obtained, D-allulose was added to each of them as a substrate such that the final concentration was 100 mM. The enzyme reaction was caused at 30°C for 4 hours, and thereafter, the generation amount of D-allose was measured to compare the generation amount of D-allose by the peak area of HPLC. Specifically, regarding the composition of the enzyme reaction solution, the reaction was caused for 4 hours at 30°C using a 50 mM Glycine-NaOH buffer (pH 9.0), 100 mM D-allulose, the crude enzyme solution, and 1 mM manganese chloride, and the reaction was stopped by boiling for 2 minutes to measure the liquid composition after the reaction by performing HPLC. Fig. 1 illustrates the peak area ratio of D-allose generated by HPLC analysis.

Regarding the conversion amount from D-allulose to D-allose when using the crude enzyme per the same culture liquid volume, the conversion amount of the present crude enzyme derived from the strain GuaL218-3 was about 23 times that of the control crude enzyme derived from the strain AgM30.

### <Experiment 5: Purification of enzyme>

### 1. Purification by ion exchange chromatography and hydrophobic chromatography

The two crude enzyme solutions were purified by ion chromatography. The column used was HiTrapQ HP equilibrated with a buffer (20 mM Tris-HCl, pH 7.5), and using an AKTA system, fractionation was caused into 5 mL fractions at the flow rate of 5 mL/min and the concentration gradient of 1 M NaCl from 0% to 100%. The fraction from which enzyme activity was detected was recovered to obtain a purified enzyme by ion exchange chromatographic separation.

2. The enzyme purified by ion exchange chromatographic separation was further purified by hydrophobic chromatography. The column used was HiTrap PHENYL, and ammonium sulfate was added to and dissolved in the enzyme solution to obtain a 2 M solution. The resulting solution was eluted by reducing the concentration of 2 M ammonium sulfate from 100% to 0% at the flow rate of 5 mL/min and fractionated into 5 mL portions. The fractions from which enzyme activity was detected were dialyzed to remove ammonium sulfate, and thereby a purified enzyme was obtained by hydrophobic chromatographic separation.

### 3. Polyacrylamide gel electrophoresis

SDS-PAGE (gel concentration 12.5%) was performed according to the conventional method to confirm the purity of the purified enzyme. In Fig. 2, a standard protein is illustrated on the left side, and the enzyme after purification using hydrophobic chromatography is illustrated on the center and right lanes. According to the results, a single band was recognized at about 48 kDa, which confirmed that the enzyme was purified completely, thereby revealing that the subunit molecular mass of the purified present enzyme by SDS-PAGE was about 48 kDa.

### <Experiment 6: Measurement of physicochemical properties of enzyme>

For the measurement of the activity of the L-rhamnose isomerase as the purified enzyme, an enzyme reaction was caused by performing the following experiment. Using 5 mM L-rhamnose as a substrate, an enzyme reaction was caused for 10 minutes under each condition. After the reaction, 50 µL of a 10% trichloroacetic acid solution was added to the reaction solution to stop the reaction, and L-rhamnulose generated was measured by a cysteine carbazole sulfuric-acid method.

### 1. Optimum reaction pH

For the measurement, the reaction was caused at 30°C for 10 minutes by using 5 mM L-rhamnose as a substrate and using various buffers having the pH of 3 to 11, and the generated L-rhamnulose was measured using the cysteine carbazole sulfuric-acid method to obtain the optimum reaction pH.

The reaction conditions are shown in Table 1. The buffers used are shown in Table 2.

**[Table 1]**

| Reaction condition | |
|---|---|
| Enzyme solution | |
| L-rhamnose | Final concentration 5 mM |
| Manganese chloride | Final concentration 1 mM |
| Buffer (pH 3.0 to 11.0) | Final concentration 50 mM |
| Reaction temperature | 30°C |
| Reaction time | 10 minutes |

**[Table 2]**

| Buffer | |
|---|---|
| Citrate | pH 3.0, pH 4.0 |
| Acetate | pH 4.0, pH 5.0, pH 6.0 |
| Phosphate | pH 6.0, pH 7.0, pH 8.0 |
| Tris-HCl | pH 7.5, pH 8.0, pH 9.0 |
| Glycine-NaOH | pH 9.0, pH 10.0, pH 11.0 |

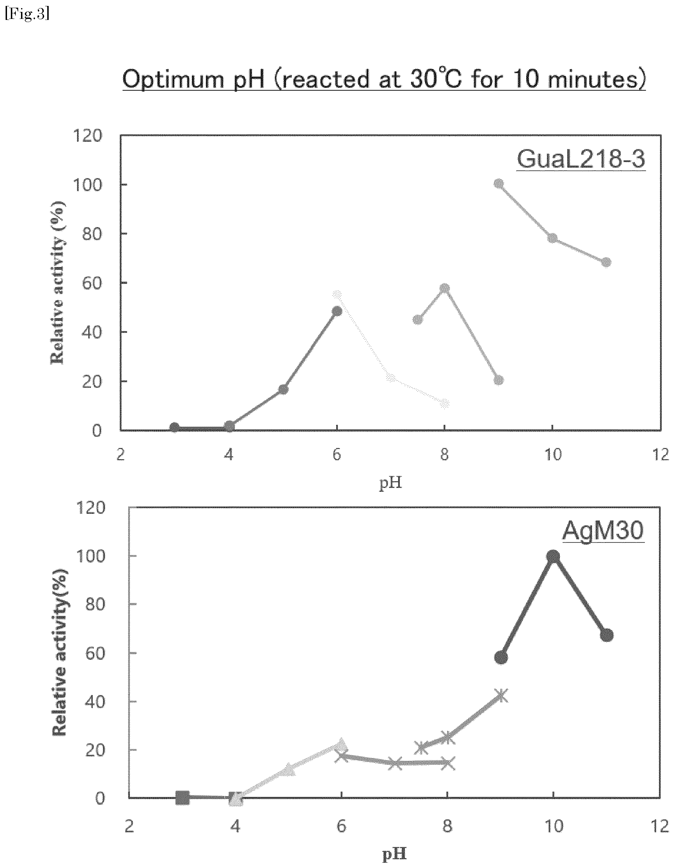

The results are illustrated in Fig. 3. The optimum reaction pH of the present enzyme was 9, whereas the optimum reaction pH of the control enzyme was 10 and was on the alkaline side. The optimum reaction pH of the present enzyme was over a wider range, showing a high activity under more acidic side.

### 2. pH stability

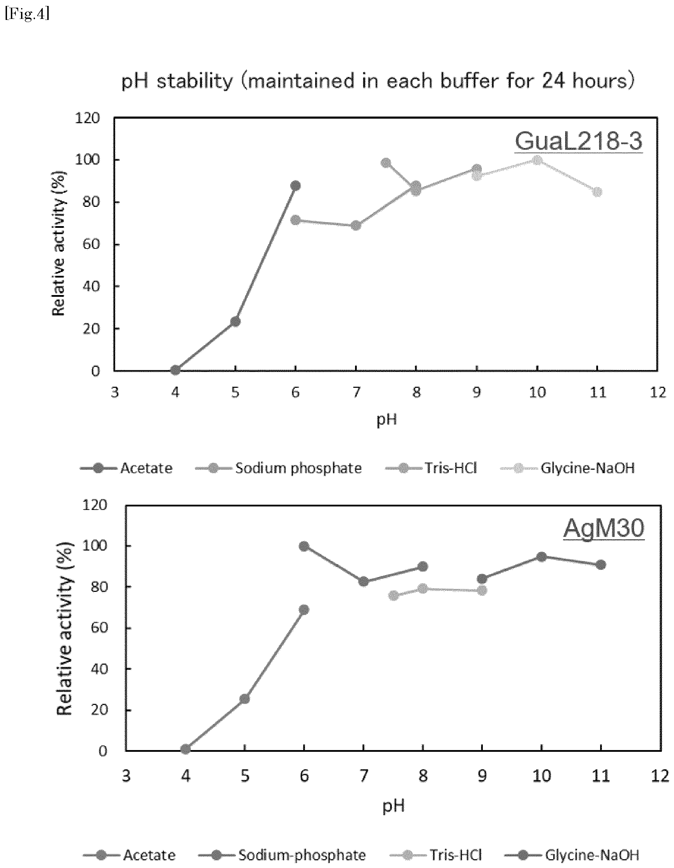

Next, Fig. 4 illustrates the residual activity after maintaining at 30°C for 24 hours in each of the buffers, using the four buffers having the pH of 4 to 11 in Table 2.

Both of the present enzyme and the control enzyme were stable at the pH of 6 to 11, but the pH stability of the control enzyme was the highest at the pH of 6, whereas the present enzyme was most stable at the pH of 7.5 and 10.

### 3. Optimum reaction temperature

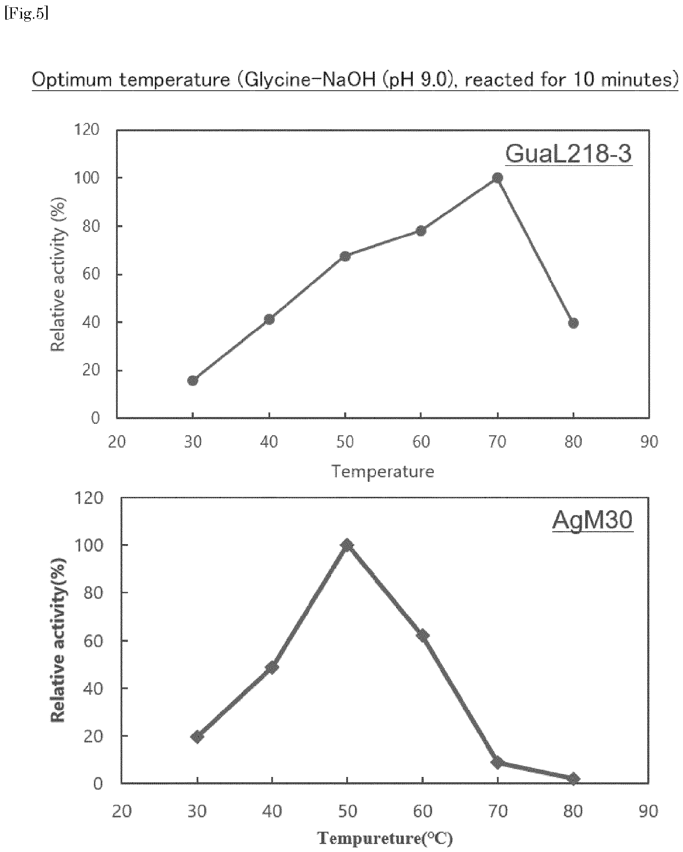

The pH was adjusted to 9 with a Glycine-NaOH buffer, and the reaction was caused at various temperatures of 30°C to 80°C to obtain the optimum temperature. The reaction conditions are shown in Table 3. The temperature range from 40°C to 80°C was a preferred temperature for the present enzyme, and it was clarified from Fig. 5 illustrating the measurement results of the reaction temperature and the relative activity that the optimum temperature of the present enzyme was 70°C.

**[Table 3]**

| Reaction condition | |
|---|---|
| Enzyme solution | |
| L-rhamnose | Final concentration 5 mM |
| Manganese chloride | Final concentration 1 mM |
| Glycine-NaOH buffer (pH 9.0) | Final concentration 50 mM |
| Reaction temperature | 30°C to 80°C |
| Reaction time | 10 minutes |

The optimum temperature of the control enzyme was 50°C, but in the case of the present enzyme, the relative activity (%) with 70°C at which the particularly highest activity was shown as 100 was 40% at 80°C, 80% at 60°C, and 70% at 50°C, whereas in the case of the control enzyme, the relative activity (%) with 50°C at which the highest activity was shown as 100 was 65% at 60°C and 8% at 70°C, meaning that the present enzyme maintained the activity particularly at high temperatures and had high thermostability.

### 4. Thermal stability

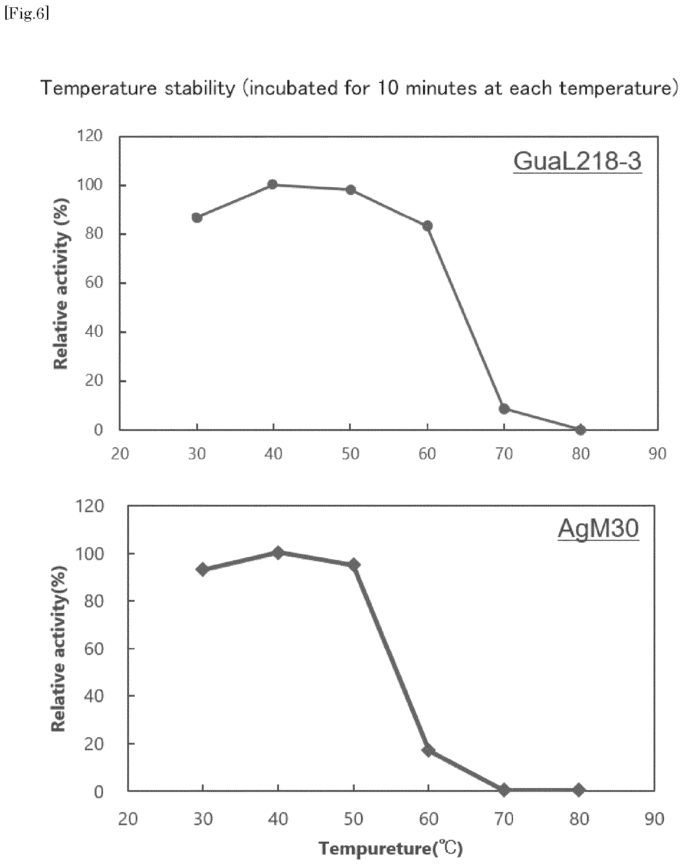

Fig. 6 illustrates the residual activity after incubation of the present enzyme and the control enzyme at each temperature for 10 minutes under the reaction conditions (10 minutes) in which the optimum temperature was obtained in the above-mentioned 3. shown in Table 3. It was found that, as compared to the control enzyme, the present enzyme was a more stable enzyme at high temperature from the results in which a decrease in the relative activity of the present enzyme at 60°C was less than 20%, which was much less than the 80% decrease of the control enzyme, in the temperature stability after the incubation for 10 minutes.

### 5. D-allose or D-allulose isomerase activity

For the measurement of the activity of the D-allose isomerase of the present enzyme, an enzyme reaction was caused by performing an experiment similar to Experiment 6. Using 100 mM D-allose as a substrate, an enzyme reaction was caused at 60°C for 60 minutes. After the reaction, the reaction was stopped by putting the reaction solution in a boiling liquid for 3 minutes to measure the liquid composition after the reaction by HPLC. One unit (U) of the enzyme activity is the amount of an enzyme that isomerizes D-allose to generate 1 µmοl of D-allulose per minute under the above-mentioned conditions. The reaction conditions are shown in Table 4.

The D-allulose isomerase activity was measured under similar conditions. Using 100 mM D-allulose as a substrate, an enzyme reaction was caused at 60°C for 60 minutes to generate D-allose. One unit (U) of the enzyme activity is the amount of an enzyme that isomerizes D-allulose to generate 1 µmοl of D-allose per minute under the above-mentioned conditions.

**[Table 4]**

| Reaction condition | |
|---|---|
| Enzyme solution | |
| D-allose | Final concentration 0.1 M |
| Manganese chloride | Final concentration 1 mM |
| Glycine-NaOH buffer (pH 9.0) | Final concentration 50 mM |
| Reaction temperature | 60°C |
| Reaction time | 60 minutes |

As a result, the specific activity of the present enzyme for D-allose was found to be 5.21 U/mg, and the specific activity for D-allulose was found to be 2.26 U/mg.

In addition, when D-allulose was isomerized to D-allose by the present enzyme, D-altrose which is a by-product was not generated.

### 6. Substrate specificity of L-rhamnose isomerase

The isomerization activity of the present enzyme for L-rhamnose and five types of aldoses (D-allose, L-talose, L-lyxose, D-ribose, and L-mannose) was examined. For the enzyme reaction composition, a reaction was caused at 70°C for 10 minutes using each substrate having the final concentration of 5 mM, and the enzyme solution (phosphate buffer having the final concentration of 50 mM and the pH of 8.0) as shown in Table 5 below, and a ketose isomerized from each aldose was measured by HPLC analysis.

With the isomerization activity for L-rhamnose as 100, the activity for each aldose is shown as a relative activity.

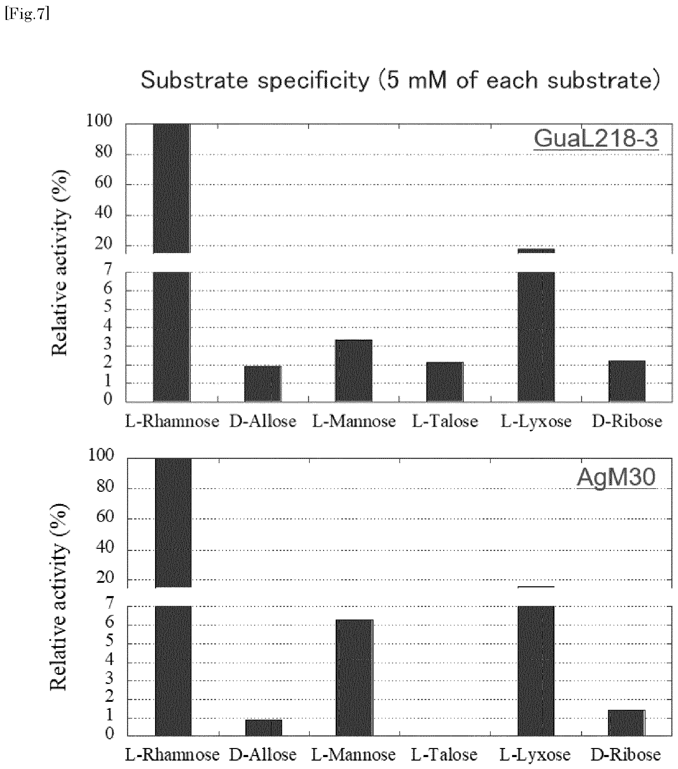

The activity is shown as the relative activity in Table 6 and Fig. 7 using D-allose, L-mannose, L-talose, L-lyxose, and D-ribose as substrates.

**[Table 5]**

| Reaction condition | |
|---|---|
| Enzyme solution | |
| Substrate | Final concentration 5 mM |
| Manganese chloride | Final concentration 1 mM |
| Glycine-NaOH buffer (pH 9.0) | Final concentration 50 mM |
| Reaction temperature | 70°C |
| Reaction time | 10 minutes |

**[Table 6]**

| Substrate | Relative activity (%) |
|---|---|
| L-rhamnose | 100 |
| L-lyxose | 17.9 |
| L-mannose | 3.3 |
| D-ribose | 2.2 |
| L-talose | 2.2 |
| D-allose | 1.9 |

The activity for L-rhamnose was the strongest, followed by L-lyxose, L-mannose, D-ribose, L-talose, and D-allose in this order. On the other hand, in the case of the control enzyme, the order was L-rhamnose, L-lyxose, L-mannose, D-ribose, and D-allose, and there was no reaction with L-talose. The present enzyme catalyzes the isomerization reaction between L-rhamnose and L-rhamnulose, between L-lyxose and L-xylulose, between L-mannose and L-fructose, between D-ribose and D-ribulose, between L-talose and L-tagatose, and between D-allose and D-allulose.

### 7. Influence of metal ions

Next, the enzyme was partially dialyzed to measure the enzyme activity for the purpose of examining the influence of metal ions on the D-allose isomerase activity. Dialysis was performed by putting the enzyme solution in a cellulose membrane, immersing it in a Glycine-NaOH buffer (pH 9.0) containing 20 mM EDTA, and slowly stirring this buffer for 16 hours, and the influence of other metal ions was removed. The enzyme activity of the enzyme thus obtained was measured by the cysteine carbazole method after reaction in the presence of 1 mM of various divalent metal ions (under reaction conditions in Table 7).

As a result, CoCl₂ significantly increased the activity of the present enzyme, and the present enzyme showed metal dependency (Fig. 7).

**[Table 7]**

| (Reaction condition when confirming influence of metal ions) | |
|---|---|
| Reaction condition | |
| Buffer (Gly-NaOH) | : 350 µL |
| Substrate (50 mM L-rhamnose) | : 50 µL |
| Dialysis enzyme | : 50 µL |
| Metal salt (10 mM) | : 50 µL |
| Reaction temperature | : 60°C |
| Reaction time | : 10 minutes |

### <Experiment 7: Preparation of immobilized enzyme>

The bacterial cells of the Erwinia billingiae strain GuaL218-3 which were cultured and recovered in the same manner as in Experiment 2 were used.

### 1. Acquisition of crude enzyme by ultrasonic treatment of bacterial cells

The bacterial cells in 1 L of the culture liquid were suspended in 40 mL of a 50 mM Glycine-NaOH buffer (pH 9.0), and cells were disrupted with an ultrasonic homogenizer while the resulting bacterial cell suspension was cooled in ice water. The disrupted product was centrifuged at 15,000 x g for 30 minutes, and the centrifugation supernatant thus obtained was used as a crude enzyme solution.

### 2. Immobilization of crude enzyme

The crude enzyme solution obtained above was added to an ion exchange resin or a synthetic adsorbent washed sufficiently with pure water, caused to swell, and thereafter equilibrated with a 50 mM Glycine-NaOH buffer (pH 9.0), and a crude enzyme protein was bound thereto by gently mixing for 20 hours at 4°C. Subsequently, washing with a 50 mM Glycine-NaOH buffer (pH 9.0) was performed to obtain an immobilized enzyme.

As an immobilization carrier, strongly basic anion exchange resins (SA10A, SA11A, NSA100, SA20A, PA306S, PA308, PA312, PA316, PA408, PA412, and PA418 which are manufactured by Mitsubishi Chemical Corporation), weakly basic anion exchange resins (WA10, WA20, WA21J, and WA30 which are manufactured by Mitsubishi Chemical Corporation, and FPA54, FPA60CL, and FPA95 which are manufactured by Organo), or synthetic adsorbents (XAD7HP and XAD118ON which are manufactured by Organo) were used.

### 3. Method of measuring enzyme activity of immobilized enzyme

The enzyme activity of the immobilized enzyme was measured by measuring the generation amount of D-allose when an enzyme reaction was caused with D-allulose as a substrate.

First, a solution (500 µL) of an immobilized enzyme resin (100 mg), a Glycine-NaOH buffer (pH 9.0) (final concentration 50 mM), manganese chloride (final concentration 1 mM), and D-allulose (final concentration 100 mM) was made to have a reaction solution composition, and the reaction was caused for 24 minutes in a constant-temperature water bath of 30°C. Thereafter, the reaction mixture was boiled at 100°C for 2 minutes to immediately stop the reaction. The solution after the reaction was cooled to room temperature, and then desalted with an ion exchange resin (mixed resin of 200CT and IRA67 (both manufactured by Organo)), and then further subjected to filter treatment to obtain an analysis sample. The analysis was conducted by measuring the peak area of the generated D-allose using high-performance liquid chromatography (column: GL-C611 (Hitachi), temperature: 60°C, eluent: 0.1 mM NaOH, flow rate: 1.0 mL/min, detector: RID-20A (Shimadzu)).

### 4. Comparison in enzyme activity among carriers used for enzyme immobilization

The enzyme activities of the present enzyme immobilized on each carrier described in the above-mentioned 2. were compared to confirm the influence of a carrier used for enzyme immobilization. A relative value was obtained by calculation supposing that the activity of a sample having the highest activity expression rate among comparison samples was 100.

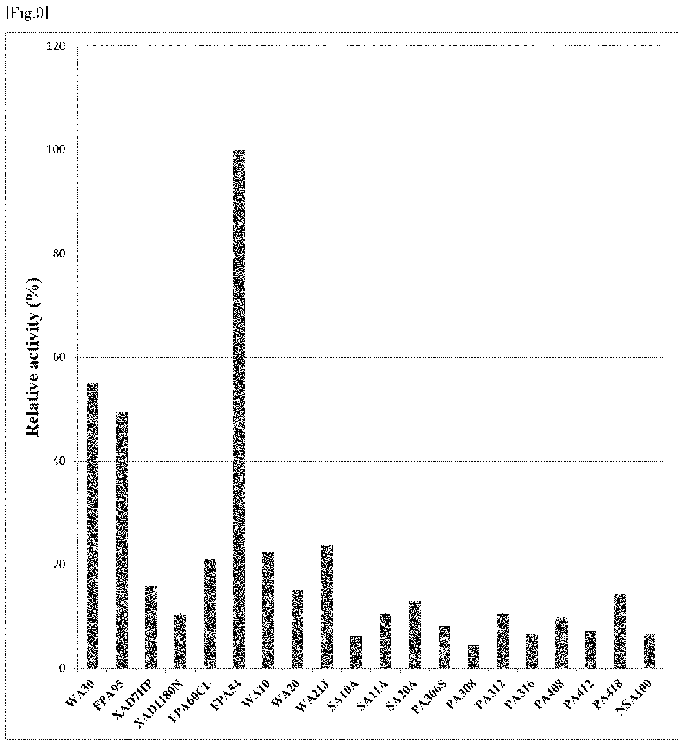

Fig. 9 illustrates relative values of the activity expression rates of various immobilized enzymes.

### <Experiment 8: Cloning of DNA encoding enzyme, and preparation of recombinant vector and transformed host cells containing the same>

A DNA encoding a protein having D-allulose isomerase activity from the Erwinia billingiae strain GuaL218-3 was cloned from the Erwinia billingiae strain GuaL218-3 to perform production of an autonomously replicable recombinant DNA, determination of the nucleotide sequence of the DNA encoding the enzyme, and preparation of a transformed microorganism.

### <Experiment 8-1: Determination of complete base sequence of chromosomal DNA>

Unlike the existing similar enzyme, the D-allose isomerase enzyme gene of this bacteria could not be isolated by using a PCR amplification method or existing protein database. The complete genome sequence of the Erwinia billingiae strain GuaL218-3 was therefore determined, and database of a protein group encoded by all ORFs in the genome sequence was constructed. The cultured bacterial cells of the Erwinia billingiae strain GuaL218-3 were requested to Macrogen Japan Corporation as the test samples, and next-generation sequence analysis was performed by using PacBio-RSII/Sequel.

As a result, four contigs with base numbers 4,301,131 bp, 1,024,754 bp, 280,568 bp, and 126,938 bp were obtained. Since these four contigs cover about 5.7 Mb, it was thought that the entire genome sequence of the Erwinia billingiae strain GuaL218-3 could be covered.

### <Experiment 8-2: Construction of protein database>

Based on the obtained DNA sequence of about 5.7 Mb, the deduced amino acid sequences of 5,357 ORFs were estimated by the Prokka program. The 5,357 amino acid sequence was used as the protein database of the Erwinia billingiae strain GuaL218-3.

### <Experiment 8-3: Identification of protein having D-allulose isomerase activity>

Using the above-described protein database, the protein was registered in the protein identification system, MASCOT server (Matrix Science), to perform identification of a protein in which D-allulose isomerase activity was observed. As a sample for the test, a 48 kDa band of SDS-PAGE in the paragraph [0045] was used, and after reduction treatment and alkylation treatment, a trypsin-digested fragment was subjected to MALDI-TOF-MS analysis.

As a result, in the amino acid sequence of sequence 1 (SEQ ID NO: 1 of Sequence Listing) consisting of the following 418 amino acids, 78% similarity with matching the underlined amino acid sequence was found, strongly suggesting that the present protein was the L-rhamnose isomerase of the Erwinia billingiae strain GuaL218-3. 
[Sequence 1]

MTKLIEQAYELAKQRYADVGIDVEQVMTQLDGIPVSMHCWQGDDVRGFENPN

GELTGGIQATGNYPGRARNAHELRADIEKAMSLIPGAKRLNLHAIYLESDTPVERDAIEP
 
QHFSHWVEWAKQHKLGLDFNPSCFSHPLSADGFTLSHADKGIRQFWIDHCKASRRISAH
FGEQLGTPSVMNIWVPDGMKDLTIDRLAPRERLASALDEVISEKFDASHHIDAVESKLFG
IGAESYTVGSNEFCLGYAASRQTALTLDAGHFHPTEVISDKISTAMLYVPRLLLHVSRPV
RWDSDHVVLLDDETQAIATEIARNKLFNKVHIGLDFFDASINRIAAWVIGTRNAKKALL
RALLEPSDQLRKLELEGDYTARLALLEEQKSLPWQAVWEAWCLRHDVPADASWLSEV
RHYEQQTLRLR
 (Sequence 1: an amino acid sequence identified using the protein database of the Erwinia billingiae strain GuaL218-3, which is a sequence in which the amino acid sequence matched the peak obtained by MALDI-TOF-MS analysis at the underlined positions)

### <Experiment 8-4: Isolation of enzyme gene having D-allulose isomerase activity>

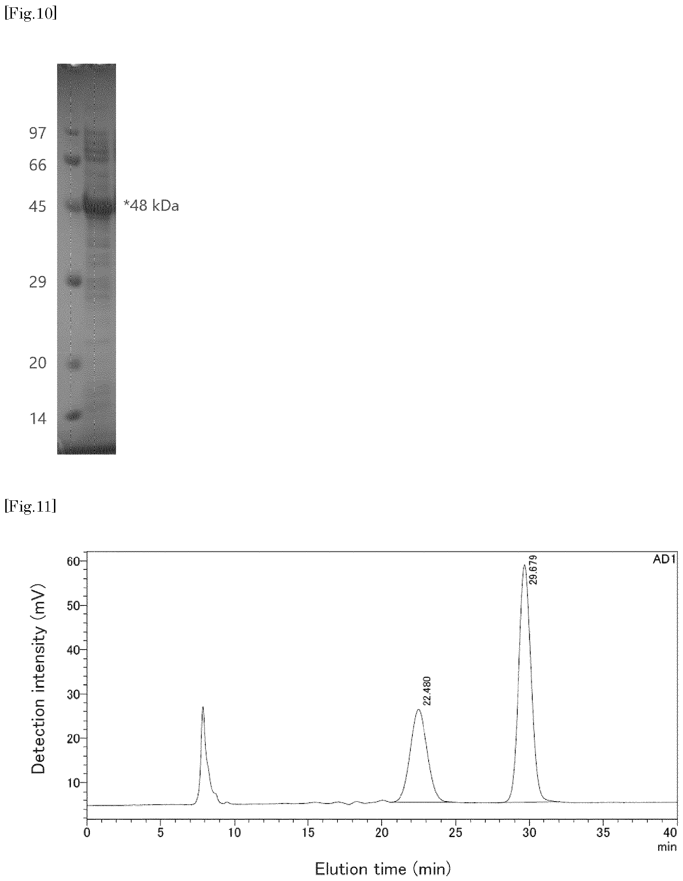

The DNA sequence of the sequence 2 (SEQ ID NO: 2 of Sequence Listing) identified from the amino acid sequence was synthesized and inserted into a pQE60 vector (Qiagen) to transform Escherichia coli for expression. The Escherichia coli expression system thus constructed was used to confirm an induced enzyme. As shown in Fig. 10, in the results of SDS-PAGE, an induced protein was confirmed in a soluble fragment. In addition, Fig. 11 shows the results of reacting 60% (w/v) D-allulose with a substrate at 30°C for 24 hours using this induced recombinant enzyme to confirm the D-allulose isomerase activity by HPLC. The peak with the retention time of 22.48 minutes was D-allose, and the peak with the retention time of 29.67 minutes was D-allulose, meaning that the present recombinant enzyme catalyzed the isomerization reaction from D-allulose to D-allose, but D-altrose, which had been a concern as a by-product, was not generated.

[Sequence 2]
ATGACAAAGCTGATTGAACAAGCTTATGAACTGGCTAAACAACGTTACGCT
GACGTAGGAATTGATGTGGAGCAGGTGATGACGCAATTGGATGGGATTCCTGTGTC

GATGCACTGCTGGCAGGGGGATGACGTGCGCGGCTTCGAAAACCCCAATGGCGAAC
TCACCGGCGGGATTCAGGCCACAGGTAACTATCCGGGACGCGCGCGTAATGCGCAT
GAACTGCGCGCCGATATCGAAAAAGCCATGTCGCTGATTCCAGGCGCTAAGCGCCT
CAATCTGCACGCCATCTATCTGGAAAGCGACACGCCGGTAGAACGTGATGCTATCG
AACCGCAACACTTCAGCCACTGGGTGGAGTGGGCAAAACAGCATAAATTAGGGCTC
GATTTTAATCCAAGCTGCTTCTCGCATCCTTTAAGTGCCGATGGTTTTACCCTGTCAC
ACGCTGACAAAGGCATCCGCCAGTTCTGGATTGATCACTGCAAGGCCAGTCGCCGC
ATCTCGGCACATTTTGGTGAACAACTGGGCACGCCCTCAGTGATGAACATCTGGGTT
CCGGACGGTATGAAAGATCTCACCATTGATCGCCTGGCGCCGCGTGAACGTTTAGCC
AGCGCGCTGGATGAAGTGATCAGCGAAAAATTCGATGCCAGTCATCATATCGACGC
CGTCGAAAGTAAGTTATTCGGGATTGGTGCTGAGAGTTATACCGTGGGGTCCAATGA
GTTTTGCCTTGGCTACGCTGCCAGCCGCCAGACCGCGCTGACACTTGATGCCGGGCA
TTTCCATCCCACTGAAGTGATCTCCGACAAAATCTCCACCGCGATGCTGTATGTCCC
GCGCCTGTTATTGCACGTCAGCCGTCCGGTGCGTTGGGACAGCGACCATGTGGTGCT
GCTGGATGATGAAACCCAGGCCATTGCCACTGAAATTGCGCGTAACAAGCTGTTCA
ACAAAGTGCATATCGGCCTCGACTTCTTTGATGCTTCCATCAACCGCATCGCGGCGT
GGGTGATTGGTACCCGCAATGCCAAAAAGGCGTTACTGCGTGCGCTGTTGGAGCCG
AGTGACCAACTGCGCAAACTGGAGCTTGAGGGGGATTACACCGCACGTCTGGCGCT
GCTGGAGGAGCAAAAGTCATTGCCATGGCAGGCTGTCTGGGAAGCCTGGTGCTTGC
GTCACGATGTCCCTGCTGATGCCAGTTGGCTCAGCGAAGTCCGTCATTATGAACAAC
AAACACTGCGTCTACGTTAA

### <Experiment 9: Construction of amino acid-substituted mutant of present enzyme, and enzyme activity and heat resistance thereof>

A phylogenetic tree was built from the amino acid sequence of the L-rhamnose isomerase set including the sequence of SEQ ID NO: 1 of the present invention and the homologous proteins in database with the method described in Patent Document 8. By comparing the amino acid sequence encoded by the DNA sequence classified into the same clade with the DNA sequence of SEQ ID NO: 2 of the present invention with the amino acid sequence of SEQ ID NO: 1, and the various L-rhamnose isomerase mutants with the amino acid mutation corresponding to the site different from those in SEQ ID NO: 1 were constructed by the sitedirect mutagenesis. The amino acid-substituted mutants of the present invention exceptionally included a mutant in which one amino acid residue (G) had been added to the amino acid of the 418th position of the C-terminal.

First, a single amino acid-substituted mutant was constructed by site-directed mutagenesis. PCR primers for both DNA strands having a nucleotide sequence obtained by adding base substitution to one or two base pairs to cause amino acid substitution at the intended mutagenesis site were synthesized, and a PCR reaction was performed using a plasmid DNA to be subjected to mutagenesis and these both primers. The amplified PCR fragments were transformed into the host Escherichia coli cells, the plasmid DNA was extracted from the transformant, and introduction of intended mutation in the resulting plasmid was confirmed by sequence analysis. A multiple substituted mutant was constructed by repeating the above-mentioned method.

Recombinant Escherichia coli expressing a recombinant enzyme containing a site-directed mutant enzyme was precultured for 12 hours at 30°C and 200 rpm in a culture medium containing 3.5% polypeptone, 2.0% yeast extract, 1.0% sodium chloride, and 2 mM manganese chloride with ampicillin added at a final concentration of 100 µg/ml. 1/20 volume of the preculture solution was inoculated into a culture medium with ampicillin added at a final concentration of 100 µg/ml for expression, and main culture was performed at 30°C and 200 rpm for 2 hours. After the main culture, IPTG was added such that the final concentration was 0.1 mM to induce expression of the recombinant enzyme overnight at 30°C and 200 rpm.

Table 8 shows the composition of the culture medium for recombinant Escherichia coli expression.

**[Table 8]**

| Culture medium composition | |
|---|---|
| Polypeptone | 3.5% |
| Yeast extract | 2.0% |
| NaCl | 1.0% |
| MnCl₂ | 2 mM |

Regarding the enzyme activities of the wild-type enzyme obtained by recombination and the crude enzyme of the amino acid-substituted mutant, the reaction was caused at each temperature for 10 minutes using a Tris-HCl buffer (pH 8.0) at the final concentration of 50 mM as a buffer with D-allulose at the final concentration of 100 mM as a substrate, the amount of D-allose produced was measured using the sugar composition in the reaction solution by HPLC.

In addition, as the residual activity of these crude enzymes after incubation at 60°C for 1 hour, the enzyme solution was heat-treated at 60°C for 1 hour, and an enzyme reaction was caused thereafter at 60°C for 10 minutes with the reaction composition shown in Table 9 to measure the residual activity.

The composition of the reaction solution is shown in Table 9 below.

**[Table 9]**

| Reaction composition | |
|---|---|
| Crude enzyme | |
| Tris-HCl buffer (pH 8.0) | 50 mM |
| D-allulose | 100 mM |
| Total | 400 µL |

The optimum reaction temperature and the relative activity of each mutant were measured by the above-mentioned method, to select amino acid-substituted mutants having a higher D-allulose isomerase activity than that of the wild-type enzyme at the optimum reaction temperature of each mutant.

The wild-type enzyme was a recombinantly produced crude enzyme, and had the optimum temperature for D-allose production of 60°C, which was lower than the optimum temperature of 70°C for L-rhamnose of the purified enzyme, but there was also a mutant of which the optimum temperature was restored by insertion of mutation.

As a result, 13 mutants having a higher optimum temperature or a higher D-allulose isomerase activity at the optimum temperature than the wild-type enzyme were obtained (Table 10).

In addition, as indices of heat resistance, the D-allulose isomerase activity ratio (T70/T50) of each mutant at the reaction temperature of 70°C and 50°C, the residual activity after incubation at 60°C for 1 hour, and the D-allulose isomerase activity ratio (T80/optimum temperature) at the reaction temperature of 80°C and the optimum temperature were measured in the same manner as the measurement of the optimum reaction temperature and the relative activity of each of the above-mentioned mutants. In addition, mutants in which any of indices was higher than that of the wild-type enzyme were selected (Tables 11 to 13).

As a result, 56 mutants in which any of the above-mentioned four indices was higher than that of the wild-type enzyme were acquired. The amino acid sequences of each of these 56 mutants are set forth in SEQ ID NOs: 3 to 8, 10 to 28, and 30 to 60 in the Sequence Listing. The mutants having the amino acid sequences of SEQ ID NOs: 9 and 29 has the activity, but all of the four indices did not reach those of the wild-type enzyme.

The mutants having the amino acid sequences of SEQ ID NOs: 3 to 60 in the Sequence Listing are respectively designated as the "sequences 3 to 60".

The amino acid substitution sites (positions) and substituted amino acids from the wild-type enzyme (SEQ ID NO: 1) in the sequences 3 to 60 are shown below. In this case, when the amino acid substitution sites shown below and the like are different from those shown in SEQ ID NOs: 3 to 60 in the Sequence Listing, then the amino acid sequences shown in the Sequence Listing are the correct sequences. In addition, the numbers in parentheses at the end of each sequence indicate the number of amino acid substitutions.

### Sequence 3 419G (1)

4 V280I (1)
5 V387I (1)
6 L198F (1)
7 S405E (1)
8 N52E (1)
9 Q124R (1)
10 D102T (1)
11 Q359L (1)
12 K152P (1)
13 D18A, F216L, T310S, L365Q (4)
14 D18A, F216L, L365Q (3)
15 Y9F, D18A, V19I, I21V, Q25K, T28A, H73D, A77S, I79V, T103K, E106D, Q113K, H117N, Q124T, P179A, I195V, A199G, E202Q, F216L, D217N, A218P, S219A, A250S, L300I, N315H, N319D, S357T, L365S, A371G, L375M, L417Q (31)
16 Y9F, D18A, V19I, I21V, T28A, H73D, A77S, I79V, T103K, E106D, Q113K, H117N, Q124T, P179A, I195V, E202Q, F216L, D217N, A218P, S219A, A250S, L300I, N315H, N319D, S357T, L365N, A371G, L375M, L417Q (29)
17 D18A, V19I, I21V, T28A, H73D, A77S, I79V, T103K, E106D, Q113K, H117N, Q124T, P179A, I195V, E202Q, F216L, D217N, A218P, S219A, A250S, L300I, N315H, N319D, S357T, L365S, A371G, L375M (27)
18 D18A, V19I, I21V, T28A, I79V, Q113K, H117N, Q124P, I195V, E202Q, F216L, A218P, S219A, A250T, T310S, N315H, N319D, S357T, L365N, L417Q (20)
19 D18A, V19I, I21V, T28A, H73D, T103K, Q113K, H117N, Q124N, H125N, E202Q, F216L, D217N, A218P, S219Q, A250T, N315H, N319D, L365N (19)
20 D18A, V19I, I21V, T28A, H73D, T103K, Q113K, H117N, Q124N, H125N, I195V, E202Q, F216L, D217N, A218P, S219Q, A250T, N315H, N319D, S357T, L365N (21)
21 Y9F, D18A, V19I, I21V, M27L, T28G, G32R, N52Q, E54A, H73D, I79L, K81Q, T103K, E106A, A109E, Q113E, H117N, Q124N, K126Q, S134T, H171Y, I195V, E202Q, V211I, F216L, D217N, A218P, S219Q, A250T, T275A, T310N, N315Q, K316N, N319D, K320R, S357T, D358A, K362Q, L365N, E406D (40)
22 D18A, V19I, I21V, M27L, T28G, G32R, N52Q, E54A, H73D, I79L, K81Q, T103K, E106A, A109E, Q113E, H117N, Q124N, K126Q, S134T, H171Y, I195V, E202Q, V211I, F216L, D217N, A218P, S219Q, A250T, T275A, T310N, N315Q, K316N, N319D, K320R, S357T, D358A, K362Q, L365N, E406D (39)
23 Y9F, D18A, V19I, I21V, T28G, N52Q, E54A, H73D, I79L, T103K, Q113E, H117N, Q124N, S134T, H171Y, I195V, E202Q, F216L, D217N, A218P, S219Q, A250T, T275A, T310N, N315Q, N319D, S357T, K362Q, L365N, E406D (30)
24 Y9F, D18A, V19I, I21V, T28A, N52Q, E54A, H73D, I79L, T103K, Q113E, H117N, Q124N, H125N, S134T, H171Y, I195V, E202Q, V211I, F216L, D217N, A218P, S219Q, A250T, T310N, N315Q, N319D, S357T, K362Q, L365N, E406D (31)
25 D18A, V19I, I21V, T28A, N52Q, E54A, H73D, I79L, T103K, Q113E, H117N, Q124K, S134T, H171Y, I195V, E202Q, V211I, F216L, D217N, A218P, S219Q, A250S, T310N, N315Q, N319D, S357T, L365N (27)
26 D18A, V19I, I21V, V26A, T28A, G32R, N52Q, E54A, H73D, I79L, E80D, T103K, Q113E, H117N, Q124K, S134T, H171Y, I195V, E202Q, A205V, F216L, D217N, A218P, S219Q, A250T, T310N, N315Q, N319D, S357T, K362Q, L365N (31)
27 L4Q, Y9F, V19I, I21V, V26A, M27I, T28R, G32R, N52Q, E54A, H73D, A77S, E80D, T103K, Q113A, S116A, H117N, E120A, Q124E, L140M, A150S, G153T, I154V, H171Y, L193I, I195V, E202Q, A205M, F216L, D217N, A218P, S219Q, T257C, T275A, T310S, A313V, N315H, K316Q, N319D, A345T, S357T, Q359R, K362Q, L363A, L365N, A374T, S405G, E406D (48)
28 V19I, I21V, V26A, M27I, T28R, G32R, N52Q, E54A, H73D, A77S, E80D, T103K, Q113A, S116A, H117N, E120A, Q124E, L140M, A150S, G153T, I154V, H171Y, L193I, I195V, E202Q, A205M, F216L, D217N, A218P, S219Q, T257C, T275A, T310S, A313V, N315H, K316Q, N319D, A345T, S357T, Q359R, K362Q, L363A, L365N, A374T, S405G, E406D (46)
29 D18E, V19N, I21V, E24D, Q25L, T28A, G32E, E54Q, H73D, M83I, K90M, D102S, T103Q, Q113K, H117N, E120A, Q124S, H125N, K126R, S134T, P139A, L140K, K152T, R167H, H171Y, L198F, E202Q, E210A, V211I, K215Q, F216L, D217N, A218P, S219Q, A250S, T254I, T310H, N315Q, N319D, Q359R, K362R, L365S, Y369F (43)
30 K3T, L4Q, I5L, Y9W, E10D, Y16F, D18A, I21V, Q25E, V26A, M27L, T28R, G32R, I33L, R46A, N52G, E54S, R68K, H73T, I79L, K81L, A82T, M83L, A89P, T103E, E106A, D108N, A109E, E111K, Q113E, S116K, H117N, Q124A, H125N, S148A, K152D, G153E, C162V, I168V, H171Y, V186I, L193I, I195V, E202Q, A205L, S206A, V211A, F216L, A218P, S219A, C245Y, L246M, T257C, T275A, T310S, A313I, N315H, K316D, N319D, K320R, A345M, S357T, D358A, Q359E, R361K, L365A, E366N, A390M, W391Y, L393Q, H395N, V397A, D400G, A401S, S402Q, S405D, E406N, H409A, Q412E, Q413D, T414V, R416S, L417Q, 419G (84)
31 K3T, L4Q, I5L, Y9W, E10D, Y16F, D18A, I21V, Q25E, V26A, M27L, T28R, G32R, I33L, R46A, N52G, E54S, R68K, H73T, I79L, K81L, A82T, M83L, A89P, T103E, E106A, D108N, A109E, E111K, Q113E, S116K, Q124A, H125N, S148A, K152D, G153E, C162V, I168V, H171Y, V186I, L193I, I195V, E202Q, A205L, S206A, V211A, F216L, A218P, S219A, C245Y, L246M, A250T, T257C, T275A, T310S, A3131, N315H, K316D, N319D, K320R, A345M, S357T, D358A, Q359E, R361K, L365A, E366N, A390M, W391Y, L393Q, H395N, V397A, D400G, A401S, S402Q, S405D, E406N, H409A, Q412E, Q413D, T414V, R416S, L417Q, 419G (84)
32 K3T, L4Q, I5L, Y9W, E10D, Y16F, D18A, I21V, Q25E, V26A, M27L, T28R, G32R, I33L, R46A, N52G, E54S, R68K, H73T, I79L, K81L, M83L, A89P, T103E, E106A, D108N, A109E, E111K, Q113G, S116T, H117N, Q124A, H125N, S148A, K152D, G153E, C162V, I168V, H171Y, V186I, L193I, I195V, E202Q, A205L, S206A, V211A, F216L, A218P, S219A, C245Y, L246M, A250T, T257C, T275A, T310S, A3131, N315H, K316D, N319D, K320R, A345M, S357T, D358A, Q359E, R361K, L365A, E366N, A390M, W391Y, L393Q, H395N, V397A, D400G, A401S, S402Q, S405D, E406N, H409A, Q412K, Q413D, T414V, R416S, L417Q, 419G (84)
33 K3T, L4Q, I5L, Y9W, E10D, D18A, I21V, E24D, Q25E, V26A, M27L, T28R, G32R, I33L, R46A, N52G, E54S, R68K, H73T, I79L, K81L, M83L, A89P, E106A, D108N, A109E, E111K, Q113A, S116K, H117N, E120A, Q124A, H125N, K126Q, S148A, D151N, K152D, G153D, C162V, I168V, H171Y, V186I, L193I, I195V, E202Q, A205L, S206A, V211A, F216L, D217N, A218P, S219A, C245Y, L246M, A250T, T257C, T275A, T310N, A313I, N315H, K316D, N319D, K320R, A345M, S357T, D358S, Q359A, R361K, K362Q, L365E, E366N, A374V, S381T, A390M, W391Y, L393Q, H395N, V397T, D400G, A401S, S402Q, S405D, E406N, H409M, Q412K, Q413D, T414V, R416S, L417Q, 419G (90)
34 L11I, D18A, I21V, Q25E, V26A, M27L, T28R, G32R, I33L, R46A, N52E, E54S, R68K, H73G, I79L, K81Q, M83L, A89P, E106A, D108N, A109E, E111K, Q113E, S116K, H117N, Q124A, H125N, D151N, K152D, G153E, I168V, H171Y, V186I, L193I, I195V, L198F, E202Q, A205L, S206N, F216L, A218P, S219A, C245Y, L246M, A250T, T257C, T275A, V280I, T310S, A313I, N315H, K316N, N319D, K320R, A345M, S357T, D358E, K362Q, L365A, E366D (60)
35 D18A, I21V, Q25E, V26A, M27L, T28R, G32R, I33L, R46A, N52E, E54S, R68K, H73G, I79L, K81Q, M83L, A89P, I97L, E106A, D108N, A109E, E111K, Q113E, S116K, H117N, Q124T, H125N, D151N, K152N, G153E, I168V, H171Y, V186I, L193I, I195V, L198F, E202Q, A205L, S206N, F216L, A218P, S219A, C245Y, L246M, A250T, T257C, T275A, V280I, T310S, A313I, N315H, K316N, N319D, K320R, A345M, S357T, D358E, K362Q, L365A, E366D, S381C (61)
36 Y16F, D18A, I21V, Q25E, V26A, M27L, T28R, G32R, I33L, R46A, N52E, E54A, R68K, H73T, I79L, K81Q, M83L, A89P, E106A, D108N, A109E, E111K, Q113E, S116K, H117N, Q124A, H125N, D151N, K152D, G153E, I168V, H171Y, V186I, L193I, I195V, E202Q, A205L, S206D, F216L, A218P, S219A, C245Y, L246M, A250T, T257C, T275A, T310S, A313I, N315H, K316N, N319D, K320R, A345M, S357T, K362Q, L365A, E366D (57)
37 Y16F, D18A, I21V, Q25E, V26A, M27L, T28R, G32R, I33L, R46A, N52E, E54A, R68K, H73T, I79L, K81Q, M83L, A89P, E106A, D108N, A109E, E111K, Q113E, S116K, H117N, Q124A, H125N, D151N, K152D, G153E, C162V, I168V, H171Y, V186I, L193I, I195V, E202Q, A205L, S206D, F216L, D217N, A218P, S219A, C245Y, L246M, A250T, T257C, T275A, T310S, A313I, N315H, K316N, N319D, K320R, A345M, S357T, K362Q, L365A, E366D (59)
38 Y16F, D18A, I21V, Q25E, V26A, M27L, T28R, G32R, I33L, R46A, N52E, E54A, R68K, H73T, I79L, K81Q, M83L, A89P, E106A, D108N, A109E, E111K, Q113E, S116K, H117N, Q124A, H125N, D151N, K152N, G153E, I168V, H171Y, V186I, L193I, I195V, E202Q, A205L, S206A, F216L, A218P, S219A, C245Y, L246M, A250T, T257C, T275A, T310S, A313I, N315H, K316N, N319D, K320R, A345M, S357T, D358E, K362Q, L365A, E366D (58)
39 Y16F, D18A, I21V, Q25E, V26A, M27L, T28R, G32R, I33L, R46A, N52E, E54A, R68K, H73T, I79L, K81Q, M83L, A89P, E106A, D108N, A109E, E111K, Q113E, S116K, H117N, Q124A, H125N, K152N, G153E, I168V, H171Y, V186I, L193I, I195V, E202Q, A205L, S206A, F216L, A218P, S219A, C245Y, L246M, A250T, T257C, T275A, T310S, A313I, N315H, K316N, N319D, K320R, A345M, S357T, D358E, K362Q, L365A, E366N (57)
40 Y16F, D18A, Q25E, V26A, M27L, T28R, G32R, I33L, R46A, N52E, E54S, R68K, H73T, I79L, K81Q, M83L, A89P, E106S, A109Q, E111K, Q113E, S116K, H117N, Q124A, H125N, K126Q, K152N, G153K, I168V, H171Y, V186I, L193I, I195V, E202Q, A205L, S206D, F216L, A218P, S219A, C245Y, L246M, A250T, T257C, T275A, T310S, A313V, N315H, K316N, N319D, K320R, A345M, S357T, D358E, K362Q, L365A, E366S (56)
41 Y16F, D18A, Q25E, V26A, M27L, T28R, G32R, I33L, R46A, N52E, E54S, R68K, H73T, I79L, K81Q, M83L, A89P, E106S, A109Q, E111K, Q113E, S116K, H117N, Q124A, H125N, K126Q, K152N, G153K, I168V, H171Y, V186I, L193I, I195V, E202Q, A205L, S206A, F216L, A218P, S219A, C245Y, L246M, A250T, T257C, T275A, T310S, A313V, N315H, K316N, N319D, K320R, A345M, S357T, D358E, K362Q, L365A, E366S (56)
42 Y16F, D18A, I21V, V26A, M27L, T28P, G32R, I33L, R46A, N52Q, E54A, R68K, H73T, I79L, K81Q, A89P, D108N, E111K, Q113E, S116K, H117N, Q124E, K152N, G153D, H171Y, V186I, L193I, I195V, E202Q, A205L, S206A, F216L, A218P, S219A, C245Y, L246M, A250T, T257C, T275A, T310S, A313I, N315H, K316N, N319D, K320R, A345M, S357T, K362Q, L365A, E366N (50)
43 Y16F, D18A, Q25E, V26A, M27L, T28R, G32R, I33L, R46S, N52E, E54S, R68K, H73S, I79L, K81Q, S84R, A89P, E106S, A109Q, E111K, Q113E, S116K, H117N, Q124A, H125N, K126Q, K152N, G153S, I168V, H171Y, V186I, L193I, I195V, E202Q, A205L, S206A, F216L, A218P, S219A, C245Y, L246M, A250T, T257C, T275A, R282Q, T310S, A313V, N315H, K316D, N319D, K320R, A345M, S357T, D358A, Q359E, L365A, E366A (57)
44 Y16F, D18A, Q25E, V26A, M27L, T28R, G32R, I33L, R46S, N52E, E54S, R68K, H73S, I79L, K81Q, S84R, A89P, E106S, A109Q, E111K, Q113E, S116K, H117N, Q124A, H125N, K126Q, K152N, G153S, I168V, H171Y, V186I, L193I, I195V, E202Q, A205L, S206A, F216L, D217N, A218P, S219A, C245Y, A250T, T257C, T275A, R282Q, T310S, A313V, N315H, K316D, N319D, K320R, A345M, S357T, D358A, Q359E, L365A, E366A (57)
45 Y16F, D18A, Q25E, V26A, M27L, T28R, G32R, I33L, R46S, N52E, E54S, R68K, H73S, A77T, I79L, K81Q, S84R, A89P, E106S, A109Q, E111K, Q113E, S116K, H117N, Q124A, H125N, K126Q, K152N, G153S, I168V, H171Y, V186I, L193I, I195V, E202Q, A205L, S206A, F216L, D217N, A218P, S219A, C245Y, L246M, A250T, T257C, T275A, R282Q, T310S, A313V, N315H, K316D, N319D, K320R, A345M, S357T, D358A, Q359E, L365A, E366A (59)
46 Y16F, D18A, I21V, Q25E, V26A, M27L, T28R, G32R, I33L, R46A, N52G, E54A, R68K, H73T, I79L, K81L, M83L, A89P, T103E, E106A, D108N, A109E, E111K, Q113E, S116K, H117N, Q124A, H125N, K152D, G153E, C162V, I168V, H171Y, V186I, L193I, I195V, E202Q, A205L, S206A, F216L, D217N, A218P, S219A, C245Y, L246M, A250T, T257C, T275A, T310S, A313I, N315H, K316D, N319D, K320R, A345M, S357T, D358A, Q359E, K362Q, L365A, E366D (61)
47 Y16F, D18A, I21V, Q25E, V26A, M27L, T28R, G32R, I33L, R46A, N52G, E54A, R68K, H73T, I79L, K81L, M83L, A89P, T103E, E106A, D108N, A109E, E111K, Q113E, S116K, H117N, Q124A, H125N, S148A, K152D, G153E, C162V, I168V, H171Y, V186I, L193I, I195V, E202Q, A205L, S206A, F216L, D217N, A218P, S219A, C245Y, L246M, A250T, T257C, T275A, T310S, A313I, N315H, K316D, N319D, K320R, A345M, S357T, D358A, Q359E, K362Q, L365A, E366D (62)
48 Y16F, D18A, I21V, Q25E, V26A, M27L, T28R, G32R, I33L, R46A, N52G, E54A, R68K, H73T, I79L, K81L, M83L, A89P, T103E, E106A, D108N, A109E, E111K, Q113E, S116K, H117N, Q124A, H125N, S148A, K152N, G153E, C162V, I168V, H171Y, V186I, L193I, I195V, E202Q, A205L, S206A, F216L, D217N, A218P, S219A, C245Y, L246M, A250T, T257C, T275A, T310S, A313I, N315H, K316D, N319D, K320R, A345M, S357T, D358A, Q359E, K362Q, L365A, E366N (62)
49 E10D, Y16F, D18A, I21V, Q25E, V26A, M27L, T28R, G32R, I33L, R46A, N52G, E54S, R68K, H73T, I79L, K81L, M83L, A89P, T103E, E106A, D108N, A109E, E111K, Q113E, S116K, H117N, Q124A, H125N, S148A, K152D, G153E, C162V, I168V, H171Y, V186I, L193I, I195V, E202Q, A205L, S206A, V211A, F216L, D217N, A218P, S219A, C245Y, L246M, A250T, T257C, T275A, T310S, A313I, N315H, K316D, N319D, K320R, A345M, S357T, D358A, Q359E, R361K, K362Q, L365A, E366N (65)
50 E10D, Y16F, D18A, I21V, Q25E, V26A, M27L, T28R, G32R, I33L, R46A, N52G, E54S, R68K, H73T, I79L, K81L, M83L, A89P, T103E, E106A, D108N, A109E, E111K, Q113E, S116K, H117N, Q124A, H125N, S148A, K152D, G153E, C162V, I168V, H171Y, V186I, L193I, I195V, E202Q, A205L, S206A, V211A, F216L, A218P, S219A, C245Y, L246M, A250T, T257C, T275A, T310S, A313I, N315H, K316D, N319D, K320R, A345M, S357T, D358A, Q359E, R361K, K362Q, L365A, E366N (64)
51 Y16F, D18A, I21V, Q25E, V26A, M27L, T28R, G32R, I33L, R46A, N52G, E54S, R68K, H73T, I79L, K81L, M83L, A89P, T103E, E106A, D108N, A109E, E111K, Q113E, S116K, H117N, Q124A, H125N, S148A, K152N, G153E, C162V, I168V, H171Y, V186I, L193I, I195V, E202Q, A205L, S206A, V211A, F216L, A218P, S219A, C245Y, L246M, A250T, T257C, T275A, T310S, A313I, N315H, K316D, N319D, K320R, A345M, S357T, D358A, Q359E, R361K, K362Q, L365A, E366N (63)
52 E10D, Y16F, D18A, I21V, Q25E, V26A, M27L, T28R, G32R, I33L, R46A, N52G, E54S, R68K, H73T, I79L, K81L, M83L, A89P, T103E, E106A, D108N, A109E, E111K, Q113E, S116K, H117N, Q124A, H125N, K126R, S148A, D151N, K152D, G153E, C162V, I168V, H171Y, V186I, L193I, I195V, E202Q, A205L, S206A, V211A, F216L, A218P, S219A, C245Y, L246M, A250T, T257C, T275A, T310S, A313I, N315H, K316D, N319D, K320R, A345M, S357V, D358A, Q359A, R361K, K362Q, L365E, E366N (66)
53 Y16F, D18A, I21V, Q25E, V26A, M27L, T28R, G32R, I33L, R46A, N52G, E54S, R68K, H73T, I79L, K81L, M83L, A89P, T103E, E106A, D108N, A109E, E111K, Q113E, S116K, H117N, Q124A, H125N, K126R, S148A, D151N, K152N, G153E, C162V, I168V, H171Y, V186I, L193I, I195V, E202Q, A205L, S206A, V211A, F216L, A218P, S219A, C245Y, L246M, A250T, T257C, T275A, T310S, A313I, N315H, K316D, N319D, K320R, A345M, S357V, D358A, Q359A, R361K, K362Q, L365E, E366N (65)
54 E10D, D18A, I21V, E24D, Q25E, V26A, M27L, T28R, G32R, I33L, R46A, N52G, E54S, R68K, H73T, I79L, K81L, M83L, A89P, E106A, D108N, A109E, E111K, Q113A, S116K, H117N, E120A, Q124A, H125N, K126Q, S148A, D151N, K152D, G153D, C162V, I168V, H171Y, V186I, L193I, I195V, E202Q, A205L, S206A, V211A, F216L, D217N, A218P, S219A, C245Y, L246M, A250T, T257C, T275A, T310S, A313I, N315H, K316D, N319D, K320R, A345M, S357T, D358S, Q359A, R361K, K362Q, L365E, E366N, A374V, S381T (69)
55 K3T, L4Q, I5L, Y9W, L11I, D18A, V19I, I21V, V23A, Q25E, V26A, M27L, T28R, G32R, I33L, R46A, N52E, E54S, R68K, H73Q, I79L, K81Q, M83L, A89P, D102T, E106A, D108N, A109E, E111K, Q113E, S116K, Q124R, K126Q, D151N, K152P, G153E, H171Y, L193I, I195V, L198F, E202Q, A205L, S206N, F216L, A218P, S219A, C245Y, L246M, T257C, T275A, V280I, T310S, A3131, N315H, K316N, N319D, K320R, A345M, S357T, D358A, Q359L, K362Q, L365N, E366D, V387I, A390M, W391Y, L393Q, V397T, D400G, A401S, S402Q, S405E, E406N, H409T, Q412K, Q413D, T414V, R416S, L417Q, 419G (81)
56 K3T, L4Q, I5L, Y9W, D18A, V19I, I21V, Q25E, V26A, M27L, T28R, G32R, I33L, R46A, N52D, E54S, R68K, I79L, K81Q, M83L, A89P, E106A, D108N, A109E, E111K, Q113E, S116K, H117N, Q124P, K126Q, D151N, K152E, G153E, H171Y, L193I, I195V, E202Q, A205L, S206N, F216L, A218P, S219A, C245Y, L246M, A250T, T257C, T275A, T310S, A313I, N315H, K316N, N319D, K320R, A345M, S357T, D358A, K362Q, L365N, E366D, A390M, W391Y, L393Q, V397T, D400G, A401S, S402Q, S405D, E406N, H409T, Q412K, Q413D, T414V, R416S, L417Q, 419G (75)
57 K3T, L4Q, I5L, Y9W, D18A, V19I, I21V, Q25E, V26A, M27L, T28R, G32R, I33L, R46A, N52E, E54T, R68K, H73N, I79L, K81Q, M83L, A89P, E106A, D108N, A109E, E111K, Q113E, S116K, H117N, Q124G, K126Q, D151N, K152E, G153E, H171Y, L193I, I195V, E202Q, A205L, S206N, F216L, A218P, S219A, C245Y, L246M, T257C, T275A, T310S, A313I, N315H, K316N, N319D, K320R, A345M, S357T, D358A, Q359L, K362Q, L365N, E366D, A390M, W391Y, L393Q, V397T, D400G, A401S, S402Q, S405D, E406N, H409T, Q412K (71)
58 Y9W, D18A, V19I, I21V, Q25E, V26A, M27L, T28R, G32R, I33L, R46A, N52E, E54T, R68K, H73N, I79L, K81Q, M83L, A89P, E106A, D108N, A109E, E111K, Q113E, S116K, H117N, Q124G, K126Q, D151N, K152E, G153E, H171Y, L193I, I195V, E202Q, A205L, S206N, F216L, A218P, S219A, C245Y, L246M, T257C, T275A, T310S, A313I, N315H, K316N, N319D, K320R, A345M, S357T, D358A, Q359L, K362Q, L365N, E366D, A390M, W391Y, L393Q, V397T, D400G, A401S, S402Q, S405D, E406N, H409T, Q412K (68)
59 Y9W, D18A, V19I, I21V, Q25E, V26A, M27L, T28R, G32R, I33L, R46A, N52E, E54T, R68K, H73N, I79L, K81Q, M83L, A89P, E106A, D108N, A109E, E111K, Q113E, S116K, H117N, Q124G, K126Q, D151N, K152E, G153E, H171Y, L193I, I195V, E202Q, A205L, S206N, F216L, A218P, S219A, C245Y, L246M, T257C, T275A, T310S, A313I, N315H, K316N, N319D, K320R, A345M, S357T, D358A, Q359L, K362Q, L365N, E366D, A390M, W391Y, L393Q, V397T, D400G, A401S, S402Q, S405D, E406N, H409T (67)
60 K3T, L4Q, I5L, Y9W, Y16F, D18A, Q25E, V26A, M27L, T28R, G32R, I33L, R46S, N52E, E54S, R68K, H73S, I79L, K81Q, S84R, A89P, E106S, A109Q, E111K, Q113E, S116K, H117N, Q124A, H125N, K126Q, K152D, G153S, I168V, H171Y, V186I, L193I, I195V, E202Q, A205L, S206A, F216L, D217N, A218P, S219A, C245Y, L246M, A250T, T257C, T275A, R282Q, T310S, A313V, N315H, K316D, N319D, K320R, A345M, S357T, D358A, Q359E, L365A, E366P, A390M, W391Y, L393Q, V397T, D400G, A401S, S402E, S405E, E406S, H409A, Q412K, Q413E, T414I, R416S, L417R, 419G (78)

In summary, the amino acid-substituted mutants of the sequences 3 to 60 have specific amino acid substitutions at the following 122 sites.

K3T, L4Q, I5L, Y9F/W, E10D, L11I, Y16F, D18A/E, V19I/N, 121V, V23A, E24D, Q25K/L/E, V26A, M27L/I, T28A/G/R/P, G32E/R, I33L, R46A/S, N52D/E/G/Q, E54A/Q/S/T, R68K, H73D/G/N/S/T/Q, A77S/T, I79L/V, E80D, K81Q/L, A82T, M83I/L, S84R, A89P, K90M, I97L, D102S/T, T103E/K/Q, E106A/D/S, D108N, A109E/Q, E111K, Q113A/E/K/G, S116A/K/T, H117N, E120A, Q124A/E/G/K/N/R/P/S/T, H125N, K126R/Q, S134T, P139A, L140K/M, S148A, A150S, D151N, K152D/E/N/P/T, G153D/E/K/S/T, I154V, C162V, R167H, I168V, H171Y, P179A, V186I, L193I, I195V, L198F, A199G, E202Q, A205L/M/V, S206A/D/N, E210A, V211A/I, K215Q, F216L, D217N, A218P, S219A/Q, C245Y, L246M, A250S/T, T254I, T257C, T275A, V280I, R282Q, L300I, T310N/H/S, A313I/V, N315H/Q, K316D/N/Q, N319D, K320R, A345M/T, S357T/V, D358A/E/S, Q359A/E/L/R, R361K, K362Q/R, L363A, L365A/E/N/S/Q, E366A/D/N/P/S, Y369F, A371G, A374T/V, L375M, S381C/T, V387I, A390M, W391Y, L393Q, H395N, V397A/T, D400G, A401S, S402Q/E, S405D/E/G, E406D/N/S, H409A/T/M, Q412E/K, Q413D/E, T414V/I, R416S, L417Q/R, 419G

Furthermore, the amino acid-substituted mutants of the sequences 3 to 60 have amino acid substitutions and addition of one amino acid residue at the following 122 sites.

K3, L4, I5, Y9, E10, L11, Y16, D18, V19, I21, V23, E24, Q25, V26, M27, T28, G32, I33, R46, N52, E54, R68, H73, A77, I79, E80, K81, A82, M83, S84, A89, K90, I97, D102, T103, E106, D108, A109, E111, Q113, S116, H117, E120, Q124, H125, K126, S134, P139, L140, S148, A150, D151, K152, G153, I154, C162, R167, I168, H171, P179, V186, L193, I195, L198, A199, E202, A205, S206, E210, V211, K215, F216, D217, A218, S219, C245, L246, A250, T254, T257, T275, V280, R282, L300, T310, A313, N315, K316, N319, K320, A345, S357, D358, Q359, R361, K362, L363, L365, E366, Y369, A371, A374, L375, S381, V387, A390, W391, L393, H395, V397, D400, A401, S402, S405, E406, H409, Q412, Q413, T414, R416, L417, 419

Among the sequences 3 to 60, the sequence 33 has the largest number of amino acid substitutions, which is 90, and has the amino acid sequence identity of 78% with the wild-type enzyme of the sequence 1.

In addition, among 13 sequences having a higher D-allulose isomerase activity at the optimum temperature than the wild-type enzyme of the sequence 1 shown in Table 10 below, the sequence 32 has the largest number of amino acid substitutions, which is 84, and has the identity of 80% with the amino acid sequence of the sequence 1.

**[Table 10]**

| | Activity at optimum temperature (U) | Optimum temperature |
|---|---|---|
| Sequence 32 | 107.32 | 70°C |
| 46 | 104.94 | 70°C |
| 55 | 98.62 | 60°C |
| 13 | 97.09 | 70°C |
| 26 | 96.92 | 70°C |
| 15 | 96.16 | 70°C |
| 51 | 94.09 | 70°C |
| 17 | 83.77 | 70°C |
| 10 | 80.26 | 70°C |
| 21 | 80.05 | 70°C |
| 20 | 79.23 | 70°C |
| 37 | 77.32 | 60°C |
| 49 | 75.79 | 70°C |
| 1 | 75.54 | 60°C |

Table 11 shows the numbers of 52 sequences having a higher D-allulose isomerase activity ratio (T70/T50) at the reaction temperature of 70°C and 50°C than the wild-type enzyme of the sequence 1.

**[Table 11]**

| Sequence | T70/T50 |
|---|---|
| 42 | 305 |
| 27 | 207 |
| 14 | 1.98 |
| 56 | 1.91 |
| 21 | 1.90 |
| 48 | 1.87 |
| 17 | 1.84 |
| 52 | 1.83 |
| 26 | 1.83 |
| 58 | 1.82 |
| 19 | 1.80 |
| 39 | 1.79 |
| 22 | 1.76 |
| 8 | 1.75 |
| 51 | 1.69 |
| 46 | 1.69 |
| 5 | 1.67 |
| 57 | 1.67 |
| 50 | 1.66 |
| 31 | 1.63 |
| 40 | 1.63 |
| 20 | 1.62 |
| 18 | 1.61 |
| 59 | 1.60 |
| 41 | 1.59 |
| 49 | 1.59 |
| 23 | 1.58 |
| 34 | 1.56 |
| 54 | 1.55 |
| 33 | 1.55 |
| 25 | 1.55 |
| 38 | 1.50 |
| 44 | 1.48 |
| 53 | 1.47 |
| 43 | 1.46 |
| 10 | 1.44 |
| 7 | 1.43 |
| 13 | 1.42 |
| 15 | 1.41 |
| 55 | 1.40 |
| 60 | 1.40 |
| 47 | 1.36 |
| 24 | 1.35 |
| 37 | 1.33 |
| 45 | 1.30 |
| 35 | 1.29 |
| 16 | 1.28 |
| 30 | 1.27 |
| 32 | 1.21 |
| 12 | 1.21 |
| 36 | 1.19 |
| 28 | 1.17 |
| 1 | 1.16 |

Table 12 shows the numbers of 17 sequences having a higher residual activity after incubation at 60°C for 1 hour than the wild-type enzyme of the sequence 1.

**[Table 12]**

| Sequence | Residual activity when incubation at 60°C for 1 hour (%) |
|---|---|
| 41 | 115.2 |
| 22 | 113.6 |
| 46 | 113.0 |
| 36 | 112.2 |
| 47 | 110.4 |
| 6 | 110.4 |
| 34 | 109.5 |
| 28 | 107.3 |
| 30 | 107.2 |
| 40 | 107.2 |
| 27 | 107.0 |
| 42 | 105.7 |
| 19 | 105.4 |
| 17 | 104.8 |
| 51 | 104.5 |
| 21 | 103.8 |
| 49 | 103.6 |
| 1 | 103.4 |

Table 13 shows the numbers of 56 sequences having a higher D-allulose isomerase activity ratio (T80/optimum temperature) at the reaction temperature of 80°C and the optimum temperature than the wild-type enzyme of the sequence 1.

**[Table 13]**

| Sequence | Relative activity at T80/optimum temperature (%) |
|---|---|
| 45 | 92.0 |
| 51 | 83.9 |
| 56 | 83.7 |
| 43 | 82.6 |
| 33 | 81.1 |
| 46 | 79.9 |
| 41 | 79.4 |
| 17 | 76.6 |
| 48 | 75.6 |
| 31 | 73.1 |
| 38 | 71.1 |
| 22 | 68.6 |
| 21 | 68.0 |
| 36 | 67.3 |
| 37 | 66.7 |
| 52 | 66.3 |
| 32 | 66.1 |
| 50 | 63.7 |
| 44 | 60.2 |
| 39 | 59.7 |
| 49 | 59.0 |
| 20 | 58.8 |
| 55 | 58.4 |
| 53 | 56.8 |
| 47 | 52.8 |
| 30 | 52.3 |
| 23 | 49.7 |
| 59 | 49.0 |
| 10 | 48.5 |
| 19 | 47.4 |
| 13 | 44.1 |
| 28 | 43.4 |
| 8 | 41.7 |
| 26 | 40.2 |
| 42 | 39.9 |
| 40 | 38.4 |
| 16 | 37.2 |
| 58 | 35.9 |
| 14 | 35.1 |
| 54 | 34.8 |
| 34 | 34.1 |
| 5 | 33.4 |
| 18 | 32.4 |
| 6 | 31.3 |
| 15 | 30.9 |
| 57 | 30.5 |
| 25 | 30.1 |
| 60 | 28.8 |
| 12 | 24.1 |
| 7 | 24.0 |
| 24 | 21.7 |
| 3 | 19.6 |
| 27 | 17.4 |
| 35 | 14.0 |
| 4 | 13.5 |
| 11 | 8.3 |
| 1 | 7.7 |

### Industrial Applicability

The L-rhamnose isomerase of the present invention is characterized in that it has particularly high thermostability and a high activity as compared to conventional L-rhamnose isomerases derived from microorganisms. For example, a conventional L-rhamnose isomerase derived from Pseudomonas stutzeri has an optimum temperature of 60°C, whereas the enzyme of the present invention has a high optimum temperature of 70°C, and also has thermal stability in which a residual activity after heat treatment at 60°C for 10 minutes is 80% or more, which makes the enzyme suitable for use in industrial production.

In addition, when a substrate is D-allulose, the enzyme of the present invention has a conversion activity to D-allose as high as 2.26 U per mg protein at 60°C, which paves the way for mass production of D-allose by the present invention.

Furthermore, the L-rhamnose isomerase of the present invention can be obtained as an immobilized enzyme having a high activity by various immobilization methods. Using the immobilized enzyme makes it possible to continuously perform a mass isomerization reaction. Industrial immobilization makes intended mass production of an aldose possible.

Furthermore, the amino acid-substituted mutants of the L-rhamnose isomerase of the present invention include a lot of mutants having a higher enzyme activity or higher thermostability than those of the present enzyme before mutation.

Accordingly, the establishment of the L-rhamnose isomerase of the present invention and a mutant thereof, and a production method therefor have a remarkably great industrial significance not only in the sugar production industry but also in foods, cosmetics, pharmaceutical products, and agrochemical industries associated therewith.

## Claims

1. L-Rhamnose isomerase derived from a microorganism belonging to the genus Erwinia,
wherein a subunit molecular mass measured by SDS-PAGE is 48 kDa, and
the L-rhamnose isomerase has the following substrate specificities (A) and (B),
(A) an isomerase activity of recognizing and reacting with a CHO group of C1 and an OH group of C2 of an aldose to convert the CHO group of C1 to an OH group and the OH group of C2 to a CO group, alternatively, recognizing and reacting with an OH group of C1 and a CO group of C2 of a ketose to convert the OH group of C1 to a CHO group and the CO group of C2 to an OH group, and
(B) an activity of catalyzing an isomerization reaction between L-rhamnose and L-rhamnulose, between L-lyxose and L-xylulose, between L-mannose and L-fructose, between D-ribose and D-ribulose, between L-talose and L-tagatose, and between D-allose and D-allulose.

2. The L-rhamnose isomerase according to claim 1,
wherein the L-rhamnose isomerase has the following physicochemical properties (C) and (D),
(C) an optimum reaction pH is 9, and
(D) an optimum reaction temperature is 70°C.

3. The L-rhamnose isomerase according to claim 1 or 2, wherein the microorganism belonging to the genus Erwinia is Erwinia billingiae.

4. The L-rhamnose isomerase according to any one of claims 1 to 3, wherein the microorganism belonging to the genus Erwinia is Erwinia billingiae strain GuaL218-3 that is internationally deposited in the NITE Patent Microorganisms Depositary as an accession number NITE BP-03142.

5. A protein comprising an amino acid sequence set forth in SEQ ID NO: 1.

6. A protein that is an amino acid-substituted mutant of a protein consisting of an amino acid sequence set forth in SEQ ID NO: 1,
wherein the mutant has 80% or more identity with the amino acid sequence set forth in SEQ ID NO: 1, has amino acid substitution at least one site selected from K3, L4, I5, Y9, E10, L11, Y16, D18, V19, I21, V23, Q25, V26, M27, T28, G32, I33, R46, N52, E54, R68, H73, A77, I79, E80, K81, M83, A89, D102, T103, E106, D108, A109, E111, Q113, S116, H117, Q124, H125, K126, S134, S148, D151, K152, G153, C162, I168, H171, P179, V186, L193, I195, L198, A199, E202, A205, S206, V211, F216, D217, A218, S219, C245, L246, A250, T257, T275, V280, L300, T310, A313, N315, K316, N319, K320, A345, S357, D358, Q359, R361, K362, L365, E366, A371, L375, V387, A390, W391, L393, H395, V397, D400, A401, S402, S405, E406, H409, Q412, Q413, T414, R416, L417, and 419 of the amino acid sequence set forth in SEQ ID NO: 1, has the following L-rhamnose isomerase activities (A) and (B), and has a higher L-rhamnose isomerase activity at an optimum temperature than the protein consisting of the amino acid sequence set forth in SEQ ID NO: 1,
(A) an isomerase activity of recognizing and reacting with a CHO group of C1 and an OH group of C2 of an aldose to convert the CHO group of C1 to an OH group and the OH group of C2 to a CO group, alternatively, recognizing and reacting with an OH group of C1 and a CO group of C2 of a ketose to convert the OH group of C1 to a CHO group and the CO group of C2 to an OH group, and
(B) an activity of catalyzing an isomerization reaction between L-rhamnose and L-rhamnulose, between L-lyxose and L-xylulose, between L-mannose and L-fructose, between D-ribose and D-ribulose, between L-talose and L-tagatose, and between D-allose and D-allulose.

7. A protein that is an amino acid-substituted mutant of a protein consisting of an amino acid sequence set forth in SEQ ID NO: 1,
wherein the mutant has 78% or more identity with the amino acid sequence set forth in SEQ ID NO: 1, has amino acid substitution at least one site selected from K3, L4, I5, Y9, E10, L11, Y16, D18, V19, I21, V23, E24, Q25, V26, M27, T28, G32, I33, R46, N52, E54, R68, H73, A77, I79, E80, K81, A82, M83, S84, A89, K90, I97, D102, T103, E106, D108, A109, E111, Q113, S116, H117, E120, Q124, H125, K126, S134, P139, L140, S148, A150, D151, K152, G153, I154, C162, R167, I168, H171, P179, V186, L193, I195, L198, A199, E202, A205, S206, E210, V211, K215, F216, D217, A218, S219, C245, L246, A250, T254, T257, T275, V280, R282, L300, T310, A313, N315, K316, N319, K320, A345, S357, D358, Q359, R361, K362, L363, L365, E366, Y369, A371, A374, L375, S381, V387, A390, W391, L393, H395, V397, D400, A401, S402, S405, E406, H409, Q412, Q413, T414, R416, L417, and 419 of the amino acid sequence set forth in SEQ ID NO: 1, has the following L-rhamnose isomerase activities (A) and (B), and has a higher L-rhamnose isomerase activity ratio (T70/T50) at a reaction temperature of 70°C and 50°C, a higher residual activity after incubation at 60°C for one hour, or a higher L-rhamnose isomerase activity ratio (T80/optimum temperature) at a reaction temperature of 80°C and an optimum temperature, than the protein consisting of the amino acid sequence set forth in SEQ ID NO: 1,
(A) an isomerase activity of recognizing and reacting with a CHO group of C1 and an OH group of C2 of an aldose to convert the CHO group of C1 to an OH group and the OH group of C2 to a CO group, alternatively, recognizing and reacting with an OH group of C1 and a CO group of C2 of a ketose to convert the OH group of C1 to a CHO group and the CO group of C2 to an OH group, and
(B) an activity of catalyzing an isomerization reaction between L-rhamnose and L-rhamnulose, between L-lyxose and L-xylulose, between L-mannose and L-fructose, between D-ribose and D-ribulose, between L-talose and L-tagatose, and between D-allose and D-allulose.

8. A DNA encoding the protein according to any one of claims 5 to 7.

9. A DNA comprising a base sequence set forth in SEQ ID NO: 2 or a complementary sequence thereof.

10. A recombinant vector comprising the DNA according to claim 8 or 9.

11. A transformed host cell which is transformed by the recombinant vector according to claim 10.

12. Erwinia billingiae strain GuaL218-3 which is internationally deposited in the NITE Patent Microorganisms Depositary as an accession number NITE BP-03142, wherein the Erwinia billingiae strain GuaL218-3 produces the L-rhamnose isomerase according to any one of claims 1 to 4.

13. An immobilized protein, wherein the L-rhamnose isomerase according to any one of claims 1 to 4 or the protein according to any one of claims 5 to 7 is immobilized on a carrier.

14. An immobilized protein, wherein the L-rhamnose isomerase according to any one of claims 1 to 4 is immobilized on a carrier in a form of a crude enzyme present in a bacterial cell-disrupted product, or the protein according to any one of claims 5 to 7 is immobilized on a carrier in a form of a crude protein present in a bacterial cell-disrupted product of a transformed host cell.

15. The immobilized protein according to claim 13 or 14, wherein the carrier is an ion exchange resin or a synthetic adsorbent.

16. The immobilized protein according to claim 15, wherein the carrier is WA30, FPA54, or FPA95.

17. A method for producing an L-rhamnose isomerase, the method comprising culturing a microorganism of the genus Erwinia which produces the L-rhamnose isomerase according to any one of claims 1 to 4 or the transformed host cell according to claim 11 in a culture medium to accumulate and collect the L-rhamnose isomerase in a microorganism bacterial cell.

18. The method for producing L-rhamnose isomerase according to claim 17, wherein the culture medium is a mineral salt culture medium to which L-rhamnose is added.

19. A method for producing a ketose or an aldose, the method comprising causing the L-rhamnose isomerase according to any one of claims 1 to 4, the protein according to any one of claims 5 to 7, or the immobilized protein according to any one of claims 13 to 16 to act on a solution containing one or more selected from aldoses and ketoses to generate and collect a corresponding ketose or aldose.
